# EUROPEAN PATENT APPLICATION

(11) **EP 4 104 845 A1**
(43) Date of publication of application: **21.12.2022**
(21) Application number: 21180250.9
(22) Date of filing: 18.06.2021
(51) Int. Cl.: A61K 36/05, A61K 36/02, A61P 29/00

(54) **EXTRACT WITH ANTI-INFLAMMATORY EFFECT**

(71) Applicant: Medizinische Universität Innsbruck, 6020 Innsbruck (AT); MCI Management Center Innsbruck, 6020 Innsbruck (AT); ADSI - Austrian Drug Screening Institute GmbH, 6020 Innsbruck (AT)
(72) Inventor: HUBER, Lukas A., 6020 Innsbruck (AT); VALOVKA, Taras, 6020 Innsbruck (AT); GRIESBECK, Christoph, 6020 Innsbruck (AT); TROCKENBACHER, Alexander, 6020 Innsbruck (AT); LEITNER, Peter, 6020 Innsbruck (AT); BONN, Günther K., 6020 Innsbruck (AT); JAKSCHITZ, Thomas, 6020 Innsbruck (AT); GSTIR, Ronald, 6020 Innsbruck (AT)
(74) Representative: Kador & Partner PartG mbB

(57) **Abstract**

The present invention relates to an anti-inflammatory extract of soil microorganisms or a fraction thereof, wherein the microorganisms comprise one or more selected from *Chlorophyta, Xanthophyceae* or *Cyanobacteria.*

The invention further relates to compositions comprising two or more of Lyso-DGTS, p-Coumaric acid (ester-linked), oleamide, theophylline, or DGTS.

The invention further relates to pharmaceutical compositions comprising the anti-inflammatory extract of soil microorganisms or a fraction thereof or a composition comprising two or more of Lyso-DGTS, p-Coumaric acid (ester-linked), oleamide, theophylline, or DGTS as an active ingredient.

The invention further relates to an anti-inflammatory extract of soil microorganisms or a fraction thereof or a pharmaceutical composition comprising an anti-inflammatory extract of soil microorganisms or a fraction thereof or a composition comprising two or more of Lyso-DGTS, p-Coumaric acid (ester-linked), oleamide, theophylline, or DGTS as an active ingredient for use as a medicament, wherein the microorganisms comprise one or more selected from *Chlorophyta, Xanthophyceae* or *Cyanobacteria.*

The invention further relates to compositions comprising an anti-inflammatory extract of soil microorganisms or a fraction thereof, wherein the microorganisms comprise one or more selected from *Chlorophyta, Xanthophyceae* or *Cyanobacteria.*

## Description

The present invention relates to anti-inflammatory extracts of soil microorganisms or fractions thereof and their uses, in particular for use in the prophylaxis and/or treatment of inflammations or inflammatory disease or in non-pharmaceutical compositions such as in cosmetic compositions and food or drink supplement.

The invention further relates to pharmaceutical compositions comprising anti-inflammatory extracts of soil microorganisms or fractions thereof as an active ingredient, in particular for use in the prophylaxis and/or treatment of inflammations or inflammatory diseases.

The invention further relates to methods of preventing and/or treating inflammatory diseases in a subject.

The invention further relates to compositions comprising two or more of Lyso-DGTS, p-Coumaric acid (ester-linked), oleamide, theophylline, or DGTS and their uses, in particular for use in the prophylaxis and/or treatment of inflammations or inflammatory disease or in non-pharmaceutical compositions.

Inflammation can be acute or chronic with distinct characteristics. While acute inflammation is a rapid, self-limiting process, it can transform to chronic inflammation, being more insidious. Various diseases, including cardiovascular diseases, cancer, diabetes, arthritis, Alzheimer's disease, pulmonary diseases, and autoimmune diseases, are afflicted with chronic inflammation (B. B. Aggarwal and K. B. Harikumar, "Potential therapeutic effects of curcumin, the anti-inflammatory agent, against neurodegenerative, cardiovascular, pulmonary, metabolic, autoimmune and neoplastic diseases," The International Journal of Biochemistry & Cell Biology, vol. 41, no. 1, pp. 40-59, 2009). Skin injury initiates a cascade of events including inflammation, new tissue formation, and tissue remodeling which leads to wound repair. In chronic inflammation, active inflammation, tissue destruction, and attempts of repair proceed simultaneously.

The inflammatory response involves three major stages: dilation of capillaries to increase blood flow; microvascular structural changes and escape of plasma proteins from the bloodstream; and leukocyte transmigration through endothelium and accumulation at the site of injury. In addition to the defense functions, inflammatory cells are also an important source of growth factors and cytokines such as interleukin-1 (IL-1) and tumor necrosis factor-alpha (TNFα) that are necessary for cell recruitment, activation, and proliferation. However, while normal inflammation, e.g., during wound healing, is a rapid self-limiting process, deregulation of the profile and level of any of cytokines/chemokines that persists at sites of inflammation results in the development of various pathologies including cancer. The mechanisms include induction of genomic instability, alterations in epigenetic events and subsequent inappropriate gene expression, enhanced proliferation of initiated cells, resistance to apoptosis, unlimited replicative potential, tissue invasion, and metastasis.

Inflammatory responses are divided in two subsets, innate and acquired immunity. The innate immune system is a less specific and rapid reaction which plays a major role in acute inflammation or injuries. In a later phase of infections, the acquired immunity is characterized by lymphocytes carrying antigen-specific receptors. While Langerhans cells are major antigen-presenting cell-types, keratinocytes generate the expression of inflammatory cytokines and chemokines that mediate immune cells to enter the site of inflammation within the skin. By releasing pro-inflammatory agents like bioactive amines, lipid mediators, cytokines (TNFα, IL-1β, IL-6) and chemokines (CXL8 and MCP-1) an inflammatory response is initiated.

The inducible transcription factor NF-κB (nuclear factor kappa-light-chain-enhancer of activated B-cells) is an evolutionarily conserved master regulator of immune and inflammatory responses. NF-κB has been implicated in the pathogenesis of a number of inflammatory diseases, such as rheumatoid arthritis (RA), inflammatory bowel disease (IBD), multiple sclerosis, atherosclerosis, systemic lupus erythematosus, type I diabetes, chronic obstructive pulmonary disease and asthma (see S. Pai and R. Thomas, "Immune deficiency or hyperactivity-Nf-kappaB illuminates autoimmunity," Journal of Autoimmunity, vol. 31, no. 3, pp. 245-251, 2008). Since deregulated NF-κB activation is involved in various inflammatory diseases, targeting the NF-κB signaling pathway represents an attractive approach for anti-inflammatory therapies. Several categories of inhibitors have been developed to block different steps of NF-κB signaling. These include selective IKK (IκB kinase) inhibitors, proteasome inhibitors and inhibitors of the nuclear translocation. Although there are currently many inhibitors developed, many undesired side effects are occurring, leading to only a few in clinical use right now. Therefore, the current state of development is concentrating on a more direct and specific inhibitions of NF-κB.

Furthermore, NF-κB signaling pathway has been proposed to be one of the key mediators of aging. The effects of UV on the skin results from the production of ROS. Excessive free radicals activate the NF-κB signaling pathway and MAPK signaling pathway, contributing to the activation of AP-1 and NF-κB. Then it increase the level of TNF-α and the expression of MMPs, which induce the degradation of ECM and accelerated skin aging.

Glucocorticoids are used in the treatment of various skin conditions and diseases that cause damaging inflammatory reactions. Besides their beneficial effects in reducing inflammation, they have negative side effects, especially in long-term use, such as skin atrophy (see e.g. M. Ponec, "Effects of glucocorticoids on cultured skin fibroblasts and keratinocytes," International Journal of Dermatology, vol. 23, no. 1, pp. 11-24, 1984). Therefore, alternative anti-inflammatory agents are needed, and it was, accordingly, the object of the present invention to provide such agents.

It was surprisingly found that extracts obtained from one or more soil microorganisms selected from *Chlorophyta, Xanthophyceae* or *Cyanobacteria* and fractions thereof, are anti-inflammatory. Such extracts can hence be used in the prophylaxis and/or treatment of inflammations or inflammatory diseases, especially of the skin. Such extracts can be further used in non-therapeutic applications, especially for treating and/or preventing inflammatory-related conditions.

The present invention therefore provides an anti-inflammatory extract of soil microorganisms or a fraction thereof, wherein the microorganisms comprise, or consist of, one or more selected from *Chlorophyta, Xanthophyceae or Cyanobacteria.*

The extract of the invention and fractions thereof, as natural products, allow for the prophylaxis and/or treatment of inflammations and/or inflammatory diseases especially inflammatory diseases of the skin, without the negative side effects of glucocorticoids. Furthermore, they are useful for non-therapeutic applications, especially for treating and/or preventing inflammatory-related conditions.

The invention therefore further provides respective pharmaceutical compositions comprising said extract or fraction thereof as an active ingredient.

The invention therefore further provides an anti-inflammatory extract of soil microorganisms or a fraction thereof or a pharmaceutical composition comprising said extracts for use as a medicament, in particular in the prophylaxis and/or treatment of inflammations and/or inflammatory diseases, wherein the microorganisms comprise, or consist of, one or more selected from *Chlorophyta, Xanthophyceae* or *Cyanobacteria.*

Furthermore, the extract of the invention or a fraction thereof may be beneficially used in non-pharmaceutical compositions such as cosmetic compositions and food or drink supplements. The invention therefore further provides compositions comprising the anti-inflammatory extract of the invention or a fraction thereof such as a cosmetic composition or a food or drink supplement. Furthermore, the present invention provides non-therapeutic methods of treating or relieving an inflammatory-related conditions, comprising administering the anti-inflammatory extract of the invention, thereby treating or relieving of the inflammatory-related condition.

The invention further provides anti-inflammatory compositions comprising two or more of Lyso Diacylglyceryl-N,N,N-trimethylhomo-Serine (Lyso-DGTS), p-Coumaric acid (ester-linked), oleoamide (C₁₈H₃₅NO), theophyline (C₇H₈N₄O₂), and Diacylglyceryl-N,N,N-trimethylhomo-Serine (DGTS). The invention further provides respective pharmaceutical compositions comprising said anti-inflammatory compositions as an active ingredient. The invention further provides pharmaceutical compositions comprising said anti-inflammatory compositions as an active ingredient for use as a medicament, in particular in the prophylaxis and/or treatment of inflammations and/or inflammatory diseases. Furthermore, also these anti-inflammatory compositions may be beneficially used as a cosmetic composition or a food or drink supplement. Furthermore, the present invention provides non-therapeutic methods of treating or relieving an inflammatory-related conditions, comprising administering the anti-inflammatory composition of the invention, thereby treating or relieving of the inflammatory-related condition.

The invention further provides methods of preventing and/or treating inflammatory diseases in a subject the method comprising administering an anti-inflammatory extract of soil microorganisms or a fraction thereof, or a respective pharmaceutical composition comprising said extract or fraction thereof or said anti-inflammatory composition, wherein the microorganisms comprise, or consist of, one or more selected from *Chlorophyta, Xanthophyceae* or *Cyanobacteria* to a subject, in the need thereof. Preferably, the subject is a mammal, more preferably a human mammal, cats and dogs.

To identify whether an extract is anti-inflammatory and for proof thereof a test using one of several established *in vitro* models may be used and for example ELISA and multiplex technologies for cytokine measurement in inflammation may be used. Among those HaCaT cells which are a spontaneously immortalized human keratinocyte cell line from human adult (Ha) keratinocytes, cultivated under low Ca²⁺concentrations (Ca) with 37°C culture temperature (T), are one of the most often used models (see P. Boukamp, R. T. Petrussevska, D. Breitkreutz, J. Hornung, A. Markham, and N. E. Fusenig, "Normal keratinization in a spontaneously immortalized aneuploid human keratinocyte cell line," The Journal of Cell Biology, vol. 106, no. 3, pp. 761-771, 1988). For example, such a model may use HaCaT cells such as in the "Inflammatory response by genotoxic stress or by cytokine induction" test or in Cohen's d-test, which are described in detail in the methods and examples section below. Moreover, the cytokine release in reconstituted human epidermis model, as also described in the methods and examples section below detail below may also be used to identify whether an extract is anti-inflammatory.

Preferably, the extract of the invention is anti-inflammatory as shown in the reconstituted human epidermis model test, as also described in the methods and examples section under the heading "Cytokine release in Reconstituted Human Epidermis model after treatment with extracts" below.

The extract may preferably be prepared from one soil microorganism selected from *Chlorophyta, Xanthophyceae or Cyanobacteria* or from a mixture of such microorganisms or from a mixture of one or more of such microorganisms and a further microorganism different from such microorganisms.

The term "soil microorganisms" includes both eukaryotic algae and prokaryotic blue-green algae, also called cyanobacteria. Soil algae are eukaryotic, photoautotrophic microorganisms whose habitat is on the soil surface or inside the soil. They are almost always accompanied by prokaryotic cyanobacteria living in the same ecological niche. Terrestrial algae must be adapted to life on land due to at least temporarily limited water availability; in contrast, the conventional habitats of algae are all types of water bodies.

Preferably, in the extract the microorganisms comprise, or consist of, one or more of alpine soil algae or one or more of alpine soil cyanobacteria or mixtures thereof. Alpine soil algae and cyanobacteria originated from areas of alpine regions within central Europe, more preferably Austria, Switzerland and South Tyrol. Algae and cyanobacteria from the alpine region have to adapt to habitats with extreme levels of ultraviolet (UV) radiation as well as periods of insufficient light, dryness, cold and heat. Adaptation mechanisms that enable the survival in these diverse places of life may increase the presence and/or amount of metabolites. It has been found that both alpine soil algae and alpine soil cyanobacteria support the anti-inflammatory effect of the extract of the invention.

Preferably, in the extract the microorganisms comprise, or consist of, one or more microalgae or one or more microcyanobacteria or mixtures thereof.

Preferably, in the extract the algae selected from *Chlorophyta* is selected from subphylum *Chlorophytina,* more preferably is selected from class *Chlorophyceae or Trebouxiophyceae,* more preferably is selected from order *Sphaeropleales, Trebouxiophyceae, Prasiolales, Chlorellales,* more preferably is selected from family *Chromochloridaceae, Radiococcaceae, Bracteacoccaceae, Dictyococcaceae, Scenedesmaceae, Coccomyxaceae, Koliellaceae, Chlorellaceae,* more preferably is selected from genus *Pseudochlorella, Coccomyxa, Neocystis, Chromochloris, Chlorella, Dictyococcus, Muriella, Coelastrella, or Bracteacoccus.*

Preferably, in the extract the cyanobacteria is selected from class *Cyanophyceae,* subclass *Nostocophycidae,* order *Nostocales,* family Nostocaceae, genus *Nostoc.*

Preferably, in the extract the microorganisms comprise, or consist of, one or more selected from *Xanthophyceae, Nostoc sp., Pseudochlorella sp., Coccomyxa sp., Neocystis sp.; Chromochloris sp., Chlorella sp., Dictyococcus sp., Muriella sp., Coelastrella sp.,* or *Bracteacoccus sp.,* more preferably from *Xanthophyceae, Nostoc sp., Pseudochlorella subsphaerica, Coccomyxa sp., Neocystis brevis; Chromochloris sp., Chlorella saccharophila, Dictyococcus varians, Muriella sp., Coelastrella sp., or Bracteacoccus sp.*

Even more preferably, in the extract the microorganisms comprise, or consist of, one or more selected from *Coccomyxa sp., Pseudochlorella sp., Nostoc sp. or Chromochloris sp., even more preferably from Coccomyxa sp., Pseudochlorella subsphaerica, Nostoc sp. or Chromochloris zofingiensis.* More preferably, the microorganisms comprise, or consist of, *Chromochloris zofingiensis.*

The microorganisms may for example be algae or cyanobacteria such as from the ASIB 505 Culture Collection of Algae from the Botanical Institute of the University of Innsbruck (see G. Gaertner, "The culture collection of algae at the Botanical Institute of the University at Innsbruck (Austria): ie Sammlung der Algenkulturen des Institutes fur Botanik der Universität Innsbruck (Österreich)," in Berichte des naturwissenschaftlichen-medizinischen Verein, pp. 33-52, Accessed: 1985) such as microorganisms having Strain ID and/or Collection ID as indicated in Table 7 below.

The term "extract" refers to a concentrated preparation of microorganism material obtained by isolating or purifying desired active constituents with one or more extraction techniques. Examples of extraction techniques include, but are not limited to, solvent extraction, for example but not limited to extraction using water, ethanol, methanol, dichloromethane, a water/ethanol mixture, a dichloromethane/methanol mixture, chromatography and the like.

Preferably, the extract is obtainable by, more preferably obtained by
a) extraction of the whole or part of the soil microorganisms with a solvent, and
b) optionally obtaining a fraction of the product of step a).

The extraction in step a) may be done using a polar or an apolar solvent.

Solvent with a dielectric constant of more than 15 are generally considered to be polar such as ethanol, methanol, nitromethane, dimethyl formamide (DMF), acetonitrile, water or formamide. Solvents with a dielectric constant of less than 15 are generally considered to be apolar such as benzene, diethyl ether, tetrahydrofuran or dichloromethane. Mixtures of solvents may also be used.

Preferably, the solvent used in extraction step a) comprises, or consists of a solvent having a dielectric constant of between 5 to 80, more preferably of between 10 to 45, more preferably between 15 to 25 or between 30 to 40, most preferred is a solvent having a dielectric constant of between 15 to 25.

Preferably, the solvent used in extraction step a) comprises, or consists of, alcohol and/or water, or dichloromethane and/or alcohol, more preferably ethanol and/or water, or dichloromethane and/or methanol. More preferably, the solvent comprises, or consists of a mixture of ethanol and water, or a mixture of dichloromethane and methanol. Even more preferably, the solvent comprises, or consists of a mixture of 8:2 of ethanol and water by volume, or a 1:1 mixture of dichloromethane and methanol by volume. It can be assumed that a mixture of 8:2 of ethanol and water by volume extracts mostly polar compounds, while a mixture of dichloromethane/methanol 1:1 by volume extracts mostly apolar compounds. Most preferred is a mixture of 1:1 of dichloromethane and methanol by volume.

The extraction can be performed so that, first, the microorganisms to be extracted are harvested, washed, and lyophilized and optionally stored at - 20°C. Then, solvent is added and cells are disrupted for example by grinding and/or vortexing. Supernatants are transferred into new vessels after for example centrifugation, and this procedure is repeated several times such as 3 times. The collected supernatants are pooled and dried for example at room temperature. Dry extracts are stored at - 20°C until further use.

The soil microorganisms can be cultivated by several cultivation methods, such as, for example, phototrophic cultivation, or heterotrophic or mixotrophic cultivation strategies.

The most common carbon substrates used for the heterotrophic growth of the microorganisms are glucose, glycerin and acetate. In addition to carbon, hydrogen and oxygen, nitrogen is one of the most important elements in the microorganisms and accounts for 1-10% of the dry matter (see O. Perez-Garcia, F. M. E. Escalante, L. E. de-Bashan, and Y. Bashan, "Heterotrophic cultures of microalgae: metabolism and potential products," Water Research, vol. 45, no. 1, pp. 11-36, 2011). The metabolism of nitrogen and carbon is very closely linked.

As a further important energy source, light exposure has a regulatory influence on the metabolism of microorganisms and optimal growth. Due to the regulatory influence of light on the metabolism of the microorganisms, a cycle of light and darkness that mimics the natural day and night change is usually used for the optimal cultivation of the microorganisms. The selected duration of the light period has a significant influence on the biomass growth and the secondary metabolites of the microorganisms, while primary metabolic products such as proteins, carbohydrates, nucleic acids and lipids are often synthesized in the dark.

In a preferred embodiment of the invention, the soil microorganisms used for extraction have been cultivated under stress conditions. Hence, different phases of cultivation were performed, the growth phase (GP) and stationary phase (SP). While the microorganisms produced higher amounts of biomass in the GP, during the SP stress conditions were applied using modified conditions in the medium.

Such stress conditions, preferably, include one or more of the following: light stress, oxidative stress, nutrient starvation/deficiency, such as nitrogen starvation, salinity stress, glucose addition, salt addition or mixotrophic stress.

Nutrient starvation/deficiency may preferably be nitrogen starvation/deprivation which may e.g. be effected by executing a growth phase cultivation in a medium such as 3N-BBM medium (Bischoff, H. and Bold, H.C., "Phycological Studies IV. Some soil algae from enchanted rock and related algal species," in pp. 1-95) and exchanging the medium to being devoid of nitrogen such as ON-BBM for the nitrogen starvation process.

Salinity stress may e.g. be induced by supplementing the growth medium such as 3N-BBM medium with salt such as sodium chloride. Preferably, salinity stress is induced by supplementing the growth medium with 0.01 to 0.5 M salt, more preferably with 0.05 to 0.3 M salt, most preferably with 0.1 M salt. Preferably, the salt is sodium chloride. Preferably salinity stress is induced by supplementing the growth medium with 0.01 to 0.5 M sodium chloride, more preferably with 0.05 to 0.3 M sodium chloride, most preferably with 0.1 M sodium chloride.

Mixotrophic stress may e.g. be induced as a two-phase cultivation procedure, starting with the initial growth phase cultivation in a medium such as 3N-BBM medium, with a subsequent growth phase under starvation of nitrogen and phosphate, such as by exchanging the medium to being devoid of nitrogen and phosphate.

For glucose addition stress cultivation, a high amount of glucose may be added continuously to the microorganisms cultivated on a 3N-BBM medium. Preferably, glucose stress is induced by supplementing the growth medium with 0.1 wt% to 10 wt%, more preferably 2 wt% to 6 wt% glucose. Most preferably glucose stress is induced by supplementing the growth medium with 5 wt% glucose.

It has been found that cultivation under stress conditions increases the presence and/or amount of metabolites supporting the anti-inflammatory effect of the extract of the invention.

Preferably, the stress condition applied is mixotrophic stress or nitrogen starvation as described above, more preferably nitrogen starvation by exchanging the growth medium to being devoid of nitrogen such as 0N-BBM.

The extracts have been examined as to which chemical compounds are contained therein. This may be done including fractionation of a once prepared extract, for example, by preparatory liquid chromatography such as HPLC, identification of the fraction(s) which are anti-inflammatory, and identification of the chemical compounds contained therein, for example by mass spectrometry.

It was accordingly found that the extract or fraction thereof according to the invention preferably contains one or more of Lyso-DGTS, p-Coumaric acid (which may be ester-linked), oleoamide theophyline or DGTS, more preferably contains two or more of Lyso-DGTS, p-Coumaric acid (which may be ester-linked), oleoamide theophyline, or DGTS, more preferably contains at least DGTS and/or Lyso-DGTS, and most preferably contains all of the mentioned substances.

It was further found that the extracts and also the fraction thereof according to the invention preferably inhibit release of pro-inflammatory cytokines. Such pro-inflammatory cytokines are for example IL-1ra, RANTES, IL-1α, GM-CSF, TSLP. IP-10, IL-18, IL-6, IL-8, IL-10, MCP-1, IL-1β, TNFα and INF-β. Preferably the extracts or fraction thereof according to the invention inhibit release of IL-10, IP-10, MCP-1 or RANTES, or a combination thereof. Since the extract according of the invention or a fraction thereof reduced the release of pro-inflammatory cytokines, this further confirms that components in this extract or fraction thereof is anti-inflammatory.

The release of cytokines can be monitored in the reconstituted human epidermis model test, as described in the methods and examples section under the heading "Cytokine release in Reconstituted Human Epidermis model after treatment with extracts" below.

It was further found that the extracts of the invention or the fraction thereof preferably inhibits the IKK complex (Inhibitor of NF-κB Kinases) and/or the processes downstream of the IKK complex in the NF-κB pathway. Such specific inhibition of the IKK complex affects all processes downstream of the IKK complex in the NF-κB pathway as explained below. Thus, the extract of the invention or the fraction thereof preferably inhibits the processes downstream of the IKK complex in the NF-κB pathway, such as for example inhibits phosphorylation and proteasomal degradation of IkBα and ultimately inhibits NF-κB activation. Hence, the specific blockage of the IKK complex or more specifically, it's subunits preferably inhibits activation of the NF-κB pathway.

Even more preferably, the extracts of the invention or the fraction thereof inhibit subunit IKKβ of the IKK complex in the NF-κB pathway.

Preferably, processes upstream of the IKK complex in the NF-κB pathway are not affected by the extracts of the invention.

The IKK complex activity and specifically the activity of subunit IKKβ of the IKK complex can be monitored an *in vitro* immunoprecipitation kinase assay, as described in detail in the methods and examples section under the heading "Direct inhibition of IKKβ Kinase by the extracts in an *in vitro* kinase assay" below.

In the NF-κB pathway, TNFα signals through the two cell surface receptors TNFR1 (Tumor Necrosis Factor Receptor 1) and TNFR2 (Tumor Necrosis Factor Receptor 2) which, in turn, leads to the recruitment of the adaptor TNFR-associated death domain protein (TRADD) and subsequently the TGF-β activated Kinase (TAK1). TAK1 is recruited and activated through TAK1-binding protein 2 (TAB2) binding to the RIPK1 polyubiquitin chain. Upon binding the polyubiquitin chain, TAK1 phosphorylates and activates the IKK complex composed of IKKα, IKKβ and the regulatory subunit IKKγ (NEMO). IκBs are phosphorylated by the IKK complex, which results in their subsequent ubiquitination and degradation by the 26S proteasome. NF-κB is therefore released and accumulates in the nucleus, leading to binding of κB DNA consensus sequences and activation of target genes. IKK serves also as an essential component of a signaling pathway that involves activation of the Tpl2 kinase and its downstream targets, MEK1 (also named MAP2K1 - mitogen-activated protein kinase kinase 1) and ERK (extracellular signal-regulated kinases). This means that IKK controls both NF-κB and ERK activation in response to TNFα. Furthermore, activated TAK1 also phosphorylates MKKs (mitogen-activated protein kinase kinase, e.g. MKK6), leading to the activation of JNK and p38 kinase pathways.

Induced degradation of IkBα releases NF-κB, allowing it to accumulate in the nucleus, mediated by the nuclear localization signal (NLS) of Rel homology domain (RHD) proteins. In the nucleus, binding of NF-κB to the DNA specific sequences occurs, which ultimately induces the NFKBIA gene and triggers re-synthesis of the IκBα protein. This negative regulatory feedback mechanism influences the duration and severeness of the inflammatory response (see T. Valovka and M. O. Hottiger, "p65 controls NF-κB activity by regulating cellular localization of IκBβ," The Biochemical Journal, vol. 434, no. 2, pp. 253-263, 2011).

NF-κB activation requires the phosphorylation, polyubiquitination, and subsequent degradation of its inhibitory subunit, IκBα. Upstream of IκBα, the IKK complex induce the phosphorylation of IκBα. Hence, the specific blockage of the IKK complex or more specifically, it's subunits inhibits activation of the NF-κB pathway. The inhibition of IKK and specifically its subunit IKKβ may thus support the anti-inflammatory effect.

It was further found that the extract of the invention is not cytotoxic. Preferably, the extract is not cytotoxic at least at extract concentrations of equal or less than 100 µg/ml. To identify whether an extract is not cytotoxic and for proof of such an effect a resazurin assay such as described in detail in the methods and examples section under the heading "Assessment of cytotoxicity of extracts" below has been used.

It was further found that the anti-inflammatory effect of the extracts of the invention or fractions thereof is dose dependent. Preferably, the extract is anti-inflammatory at an extract concentration of equal or more than 0.05 µg/ml , more preferably of equal or more than 0.1 µg/ml, more preferably of equal or more than 1.0 µg/ml, more preferably of equal or more than 50 µg/ml. Usually, the extract concentration is less than 100 mg/ml. Dose dependency can be measured by several established methods and for example, an assay such as described in detail in the methods and examples section under the heading "Dose dependency of anti-inflammatory effect of extracts" below.

As the extract of the invention or a fraction thereof is anti-inflammatory and further in view of the above described properties, in a further embodiment of the present invention, the extract in any one of the embodiments described herein is for use as a medicament.

Accordingly, the invention provides an anti-inflammatory extract of soil microorganisms or a fraction thereof for use as a medicament, wherein the microorganisms comprises one or more selected from *Chlorophyta, Xanthophyceae, or Cyanobacteria*.

Further preferred, the extract or a fraction thereof is for use in the prophylaxis and/or treatment of inflammations and/or inflammatory diseases, preferably for inflammations and/or inflammatory diseases of the skin.

An inflammatory disease refers to a disease or disorder associated with abnormal or altered inflammation. Inflammation is a biological response initiated by the immune system as part of the healing process in response to a pathogen, damaged cells or tissues or irritants. An inflammatory disease can be either an acute or chronic inflammatory condition and can result from infections or non-infectious causes. Chronic inflammation can lead to a variety of diseases.

Inflammatory diseases include, but are not limited to allergies, asthma, rheumatoid arthritis, transplant rejection, celiac disease, chronic prostatitis, pelvic inflammatory diseases, inflammatory myopathies, atopic dermatitis, contact dermatitis, allergic reactions such as chronic allergic reactions and allergic rhinitis and conjunctivitis, inflammatory rheumatic diseases, asthma bronchiale, colitis, rheumatoid arthritis (RA), inflammatory bowel disease (IBD), multiple sclerosis, atherosclerosis, systemic lupus erythematosus, type I diabetes. Furthermore, inflammatory disease include but are not limited to diseases selected from the group consisting of (1) general or localized inflammatory disease (for example, allergies; hayfever; or granulomatosis); (2) gastrointestinal related diseases (for example ulcerative colitis, Crohn's disease, colitis, celiac sprue, pouchitis, appendicitis; gastric ulcer; duodenal ulcer; peritonitis; pancreatitis; ulcerative, acute, or ischemic colitis; cholangitis; cholecystitis, steatorrhea, hepatitis, Crone's disease; or Whipple's Disease); (3) dermal related diseases (for example, psoriasis; burns; sunburns; dermatitis; Urticarial warts or wheal); (4) respiratory diseases (for example, asthma; epiglottitis; bronchitis; emphysema; rhinitis; cystic fibrosis; interstitial pneumonitis; COPD (chronic obstructive pulmonary disease); adult respiratory distress syndrome; coniosis; alveolitis; bronchiolitis; pharyngitis; pleurisy; or sinusitis); (5) bone, joint, muscle and connective tissue related diseases (for example, eosinophilic granuloma; arthritis; arthralgia; osteomyelitis; dermatomyositis; fasciitis; Paget's disease; gout; periodontal disease; rheumatoid arthritis; myasthenia gravis; ankylosing spondylitis; or synovitis); (6) fungal infection (for example, candidiasis); or bacterial, parasitic, and similar microbial infection (for example, disseminated bacteremia; malaria; onchocerciasis; or amebiasis).

Preferably, the extract is for use in the prophylaxis and/or treatment of inflammations and/or inflammatory diseases involving NF-κB.

Preferably, the extract is used for the prevention and/or treatment of atopic dermatitis, psoriasis, contact dermatitis, allergic reactions such as chronic allergic reactions and allergic rhinitis and conjunctivitis, inflammatory rheumatic diseases, asthma bronchiale, colitis, rheumatoid arthritis (RA), inflammatory bowel disease (IBD), multiple sclerosis, atherosclerosis, systemic lupus erythematosus, type I diabetes or chronic obstructive pulmonary disease (COPD).

More preferably, the extract is used for the prevention and/or treatment of atopic dermatitis, psoriasis, contact dermatitis, allergic reactions such as chronic allergic reactions and allergic rhinitis, inflammatory rheumatic diseases, asthma bronchiale or colitis.

Even more preferably, the extract is used for the prevention and/or treatment of atopic dermatitis or psoriasis.

The extract as used in the present invention may be administered by any suitable route of administration. Preferably, the extract is prepared for and administered by systemic administration or topical administration.

Systemic administration includes oral administration, parenteral administration, transdermal administration, rectal administration, and administration by inhalation. Parenteral administration refers to routes of administration other than enteral, transdermal, or by inhalation, and is typically by injection or infusion. Parenteral administration includes intravenous, intramuscular, and subcutaneous injection or infusion. Inhalation refers to administration into the patient's lungs whether inhaled through the mouth or through the nasal passages. A topical application includes every administration that is applied to a particular place on or in the body, for example an application to body surfaces such as the skin or mucous membranes. Topical administrations include epicutaneous application, meaning that they are applied directly to the skin. For topical application range of classes including creams, foams, gels, emulsions, powders, liquid or solid preparations for inhalation, compresses, tamponades, tonsil pinhole solutions or gargle solutions lotions, sprays and ointments may be used.

The extracts according to the invention or fractions thereof are usually formulated into pharmaceutical compositions. Hence, in a further aspect, the present invention further relates to pharmaceutical compositions comprising the anti-inflammatory extract according to the invention or a fraction thereof in any one of the embodiments as described herein as an active ingredient, optionally together with a suitable carrier substance and/or excipient. Such pharmaceutical compositions comprise an effective amount of the extract according to the invention or an effective amount of a fraction thereof.

Accordingly, such pharmaceutical compositions may be for use as a medicament, preferably for use in the prophylaxis and/or treatment of inflammations and/or inflammatory diseases in any one of the embodiments as described herein.

The pharmaceutical compositions may optionally further comprise one or more additional pharmaceutically active compounds. For example, mixtures of the extract according to the invention and a fraction thereof may be used. Further, pharmaceutically acceptable excipient meaning a pharmaceutically acceptable material, composition or vehicle involved in giving form or consistency to the pharmaceutical composition may be present in the pharmaceutical compositions.

In particular, the pharmaceutical preparation may be in the form of drops, juice, syrup, tablets, lozenges, capsules, slow-release formulations, rectal or vaginal suppositories, infusions, vaporisations, disinfectant solutions, ointments, emulsions, particulate material, powders, liquid or solid preparations for inhalation, compresses, wadding, tamponades, creams, foams, gels, sprays, tonsil pinhole solutions or gargle solutions lotions. The pharmaceutical preparation may also be a dermatological preparation, preferably in the form of an application for external use.

The extract is preferably present in the pharmaceutical preparation in a concentration of equal or more than 0.05 µg/ml, preferably more than 0.1 µg/ml, more preferably more than 1 µg/ml, more preferably more than 10 µg/ml , more preferably more than 50 µg/ml. Usually, the extract is present in the pharmaceutical preparation in a concentration of below 100 mg/ml.

As the extract of the present invention or fraction thereof is anti-inflammatory, in a further embodiment of the present invention, the extract or fraction thereof as described in any one of the embodiments described herein can be used for non-therapeutic applications to provide relief of an inflammatory-related condition, particularly an inflammatory-related skin condition such as for example sunspot, reducing a decrease in skin firmness, or for age related spots.

The extract or fractions thereof may be formulated for example for cosmetic applications and applied to skin and related tissues, to provide relief of an inflammatory-related condition of the skin or may be formulated for food and drink supplements.

Accordingly, the present invention further relates to a composition, in particular a non-pharmaceutical composition, comprising an anti-inflammatory extract of soil microorganisms or a fraction thereof in any one of the embodiments as described herein, such as a cosmetic composition or a food or drink supplement.

Furthermore, the present invention relates to a use of an anti-inflammatory extract of soil microorganisms or a fraction thereof in any one of the embodiments as described herein in a non-pharmaceutical composition such as in a cosmetic composition or as a food supplement. Preferably, the use is to provide relief of an inflammatory-related condition of the skin. Furthermore, a non-pharmaceutical composition may be preferably used in research activities, e.g. as a reference or standard for example to investigate different extracts or as a solvent or carrier.

Furthermore, the present invention relates to a non-therapeutic method of treating or relieving an inflammatory-related condition, particularly an inflammatory-related skin condition, comprising administering, particularly topical administration to the skin, an effective amount of the anti-inflammatory extract of soil microorganisms or a fraction thereof as described in any one of the embodiments described herein, thereby treating or relieving of the inflammatory-related condition.

Administrations and formulations as described above for the medicament, treatments and prophylaxis can be equally used for the non-pharmaceutical applications, non-pharmaceutical compositions, non-therapeutic methods and use of an anti-inflammatory extract of soil microorganisms or a fraction thereof.

Preferably, the anti-inflammatory extract of soil microorganisms or a fraction thereof as described herein may be incorporated into a cosmetic composition suitable for topical application to skin. It may be desirable to include the anti-inflammatory extract of soil microorganisms or a fraction thereof as described herein as an ingredient in the cosmetic composition. A cosmetic composition may preferably include a dermatological acceptable carrier, the anti-inflammatory extract of soil microorganisms or a fraction thereof, and one or more optional ingredients of the kind commonly included in the particular cosmetic compositing being provided. Non-limiting examples of such optional ingredients include vitamins; peptides and peptide derivatives; and sugar amines. Other optional ingredients include sunscreen actives (or sunscreen agents) and/or ultraviolet light absorbers. In certain embodiments, the cosmetic composition may include a colorant, a surfactant, a film-forming composition, and/or a rheology modifier. Suitable cosmetic compositions herein may be in any one of a variety of forms known in the art, including, for example, an emulsion, lotion, milk, liquid, solid, cream, gel, mouse, ointment, paste, serum, stick, spray, tonic, aerosol, foam, pencil, and the like. The cosmetic compositions may also be incorporated into shave prep products, including, for example, gels, foams, lotions, and creams, and include both aerosol and non-aerosol versions. Other cosmetic compositions include antiperspirant, deodorant, and personal cleaning compositions such as soap and shampoo.

Furthermore, the present invention relates to compositions comprising two or more of Lyso-DGTS, p-Coumaric acid (ester-linked), oleoamide, theophyline, or DGTS, more preferably contains at least DGTS and/or Lyso-DGTS, and most preferably contains all of the mentioned substances. Preferably, such compositions are for use as a medicament and more preferably for use in the prophylaxis and/or treatment of inflammations and/or inflammatory diseases in any one of the embodiments as described above. Furthermore, preferably such composition may be a non-pharmaceutical composition such as in a cosmetic composition or as a food supplement as described in in any one of the embodiments as described above. Furthermore, such composition may be used in a non-therapeutic method of treating or relieving an inflammatory-related condition as described in in any one of the embodiments as described above.

Administrations and formulations as described above for the extracts can be equally used for the compositions comprising two or more of Lyso-DGTS, p-Coumaric acid (ester-linked), oleoamide, theophyline, or DGTS.

In a further aspect, the invention relates to a method of preventing and/or treating inflammations and inflammatory diseases, the method comprising administering an extractor fraction thereof or a respective pharmaceutical composition in any one of the embodiments as described herein to a subject.

In a further aspect, the invention relates to a method of preventing and/or treating inflammations and inflammatory diseases, the method comprising administering a composition comprising two or more of Lyso-DGTS, p-Coumaric acid (ester-linked), oleoamide, theophylline in any one of the embodiments as described herein to a subject.

Administrations and formulations as described above for the medicament, treatments or prophylaxis are equally applicable for the method of preventing and/or treating inflammations and/or inflammatory diseases.

Preferably, the subject is in need of such prevention and/or treatment. Preferably, the subject is a mammal, in the need thereof, more preferably a human.

In the present invention, it is to be understood that any embodiment disclosed for the anti-inflammatory extract of soil microorganisms or fraction thereof is applicable for either the pharmaceutical composition comprising an anti-inflammatory extract of soil microorganisms or fraction thereof as an active ingredient, the non-pharmaceutical composition comprising an anti-inflammatory extract of soil microorganisms or fraction thereof, the use of an anti-inflammatory extract of soil microorganisms or fraction thereof, the non-therapeutic method of treating or relieving an inflammatory-related condition, the compositions comprising one or more of Lyso-DGTS, p-Coumaric acid (ester-linked), oleamide, theophylline, or DGTS and the method of preventing and/or treating inflammations and/or inflammatory diseases. Furthermore, it is to be understood that any embodiment disclosed for the diseases is applicable for either the pharmaceutical composition comprising an anti-inflammatory extract of soil microorganisms or fraction thereof as an active ingredient, for compositions comprising one or more of Lyso-DGTS, p-Coumaric acid (ester-linked), oleamide, theophylline, or DGTS and for the method of preventing and/or treating inflammatory diseases the method comprising administering an anti-inflammatory extract of soil microorganisms or fraction thereof to a subject.

The present invention is further illustrated by the below methods and examples which refer to the following figures:

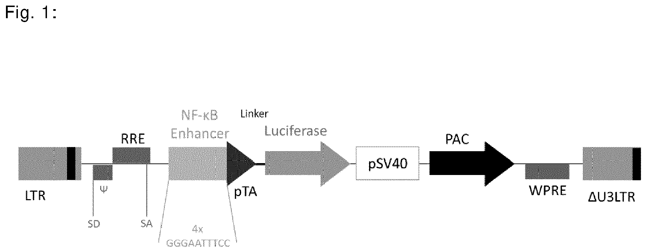

**Figure 1****: Reporter construct design:** The reporter construct consists of a firefly luciferase gene under the control of a minimal promoter (pTA) and a NF-κB enhancer element with four κB binding sites. Additional elements are long terminal repeat (LTR) sequences for genome integration, a pSV40 enhancer/promotor element, a puromycin resistance gene (PAC) and a posttranscriptional regulatory element of the woodchuck hepatitis virus (WPRE).

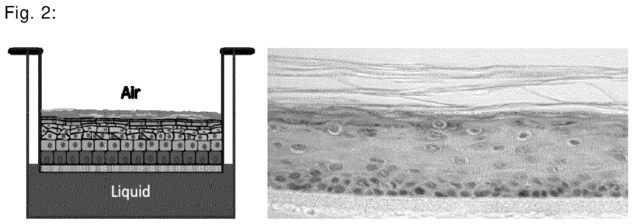

**Figure 2****: RHE model:** epiCS RHE model is reconstructed from primary human epidermal keratinocytes with a fully differentiated epidermis. Left panel shows the schematic structure in the air-liquid interface. Right panel shows epidermal layers of 3D model after Hematoxylin and Eosin staining. On the bottom the filter pores are seen and on top the air interface with the cornified layer.

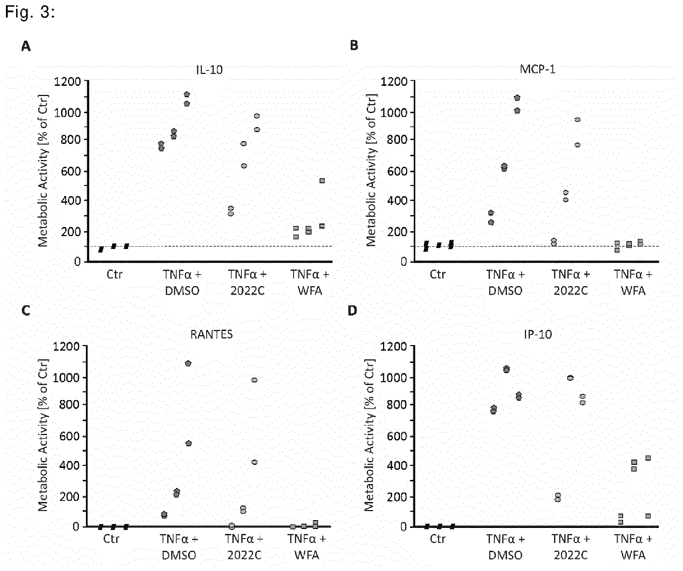

**Figure 3****: Measurement of selected cytokines in RHE models in response to TNF**α **and sample treatment:** 0.75 ng/ml TNFα was added in culture medium. IL-10 **(A),** MCP-1 **(B),** RANTES **(C)** and IP-10 **(D)** secretion in response to TNFα treatment. Untreated control (Ctr) compared to DMSO. DMSO compared to 2022C. DMSO compared to Withaferin A. Data points represent separate measurements (duplicates) of cytokines in three technical replicates. Respective position of the data points for each treatment indicate matching data from the 3 biological replicates.

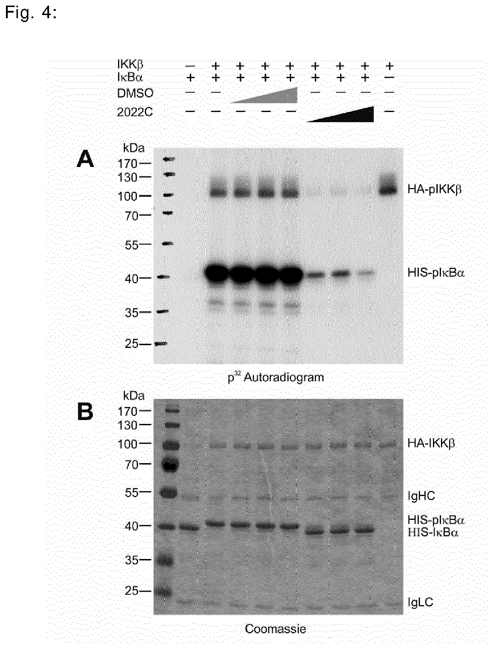

**Figure 4****: In vitro IKKβ Kinase Assay using IκBα as a substrate:** IKKβ Kinase with an N-terminal HA-tag (HA-IKKβ) was expressed in 293T cells. HA-IKKβ was then purified by immunoprecipitation using HA antibody. Kinase reaction was performed in the presence of [γ-p32]-ATP and His-IκBα. (A) Phosphorylation of IKKβ and IkBα was detected by autoradiography. (B) Total protein levels are shown by Coomassie staining. Extract 2022C was compared to the DMSO control using three different concentrations ranging from 1.6, 2.8 and 6 %.

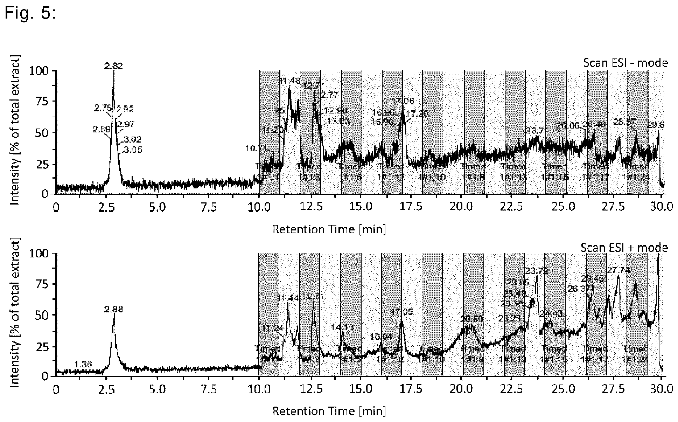

**Figure 5****: Fractionation of 2022C:** Preparative total ion chromatography of extract 2022C in negative (upper panel) and positive (lower panel) mode. The gradient profile using 0.1 % formic acid in water (solvent A) and Acetonitrile (solvent B) was as follows: 24 min from 10 % B to 90 % B, 30 min 10 % B. Flow was 3 ml/min with an injection volume of 0.5 ml. Fractions were collected after every 0.5 min starting at 10 min. Colored bar shows collected fractions and corresponding peaks in positive and negative MS-mode.

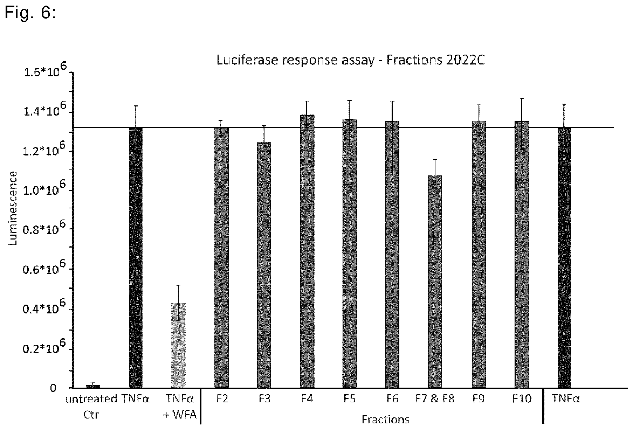

**Figure 6****: Luciferase response assay of 2022C fractions in NF-κB reporter HaCaT cells:** Absolute values of reconstituted fractions 2 to 10 are shown in comparison to TNFα (Front and Back), TNFα+WFA and untreated cells. Values are shown in relation to the induction control (black line) representative of three biological replicates. A decrease in the inflammatory response can be seen with fractions 7 and 8. These fractions are used for further investigation of active anti-inflammatory compounds. Values are mean ± SD of three measurements (*p < 0.05 and *** p < 0.001).

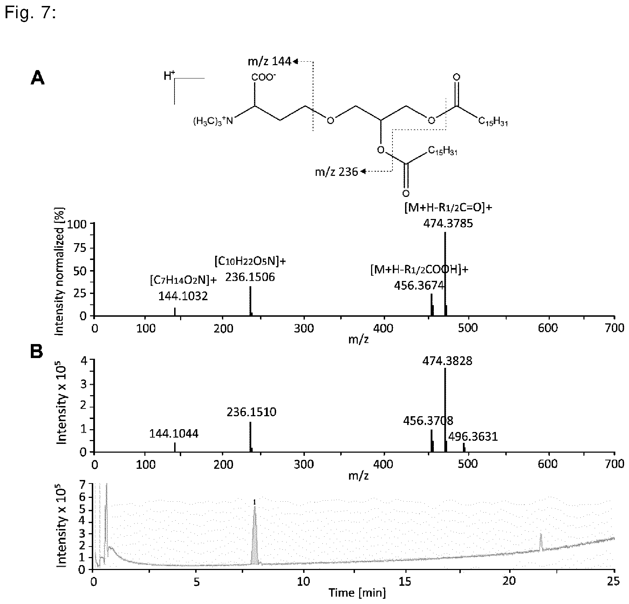

**Figure 7****: Fragmentation pattern of active fraction 8: (A)** MS/MS spectrum and fragmentation pattern of DGTS standards (16:0-16:0). **(B)** Measurement of fraction 8 with uHPLC-qTOF-MS/MS in positive mode showed predominantly the fragmentation pattern (upper panel) of the molecule DGTS.

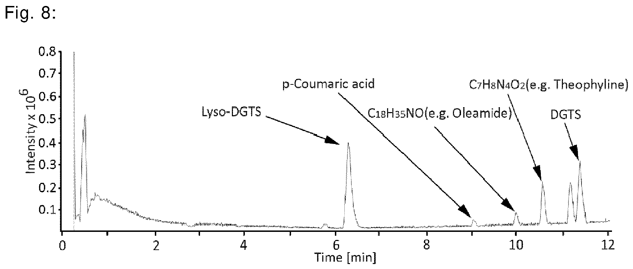

**Figure 8****: MS/MS analysis of active Fraction 8:** Identification of 5 peaks upon measurement with C18 Kinetex column using ESI+ mode. Gradient profile: 16 min from 50 % to 90 % B; 2 min from 90 % to 90 % B. Flow 0.5 ml/min with an injection volume of 10 µl.

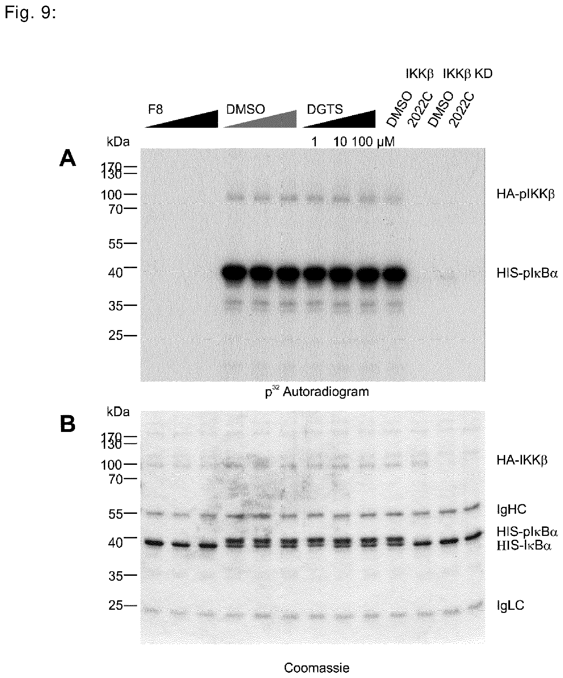

**Figure 9****: In vitro IKKβ Kinase Assay using IκBα as substrate:** IKKβ Kinase with an N-terminal HA-tag (HA-IKKβ) was expressed in 293T cells. HA-IKKβ was then purified by immunoprecipitation using HA antibody bound to Protein A Sepharose beads. Kinase reaction was performed in the presence of [γ-p32]-ATP and His-IκBα. (A) Phosphorylation of IKKβ and *IκB*α was detected by autoradiography. (B) Total protein levels are shown by Coomassie staining. Fraction 8 and DGTS were compared to the DMSO control using three different concentrations ranging from 1.6, 2.8 and 6 %.

### Examples

### Microorganism species

The microorganisms used in the experiments include algae and cyanobacteria originated from the ASIB 505 Culture Collection of Algae from the Botanical Institute of the University of Innsbruck (see G. Gaertner, "The culture collection of algae at the Botanical Institute of the University at Innsbruck (Austria): ie Sammlung der Algenkulturen des Institutes für Botanik der UniversitätInnsbruck (Österreich)," in Berichte des naturwissenschaftlichen-medizinischen Verein, pp. 33-52, Accessed: 1985). The original ASIB 505 cultures are maintained on Bold's Basal Medium (BBM) agar slants (10 g I-1) in glass test tube, which are kept at the optimal temperature of 13 °C and at a light intensity of a 300-3000 lux under cool-white fluorescent lamps on a 12 h day-night rhythm. Strains are transferred every six months to new fresh media, to avoid contamination or overgrowth by bacteria and/or fungus (see G. Gaertner, "The culture collection of algae at the Botanical Institute of the University at Innsbruck (Austria): ie Sammlung der Algenkulturen des Institutes für Botanik der UniversitätInnsbruck (Österreich)," in Berichte des naturwissenschaftlichen-medizinischen Verein, pp. 33-52).

### Microorganism cultivation and stress conditions

All procedures in preparation and maintenance of the microorganisms were performed under sterile conditions. This included the inoculation of the bioreactor, the sterile nutrient medium and the transfer of the microorganisms. The starter cultures were inoculated in shake flasks (Volume 200 ml) using 3N-BBM medium (Bischoff, H. and Bold, H.C., "Phycological Studies IV. Some soil algae from enchanted rock and related algal species," in pp. 1-95). For the subsequently performed plate cultivations, it was necessary to prepare 3N-BBM mixed with 1.5 % of Agar-Agar. In the upscaling process the optical density (OD) of the cultures were determined using photometric measurements at 750 nm. At the OD₇₅₀ of 3.0 the cultures were transferred to small-scale bioreactors (SSBR) (Volume 500 ml).

More particularly, for the cultivation the chosen microorganism strain biomass is taken from the 3N BBM agar petri dish. The biomass is directly transferred to a 500 mL small-scale bioreactor (SSBR), which could be a Schott bottle equipped with tubes allowing for aerobic culture. In the 500 mL bottles, approximately 300 mL are added and for the 2000 mL bottles, a volume of 1000 to 1300 mL is used. After adding the 3N BBM broth bottles are autoclaved at 121°C for 20 minutes. Prior to the inoculation, the broth and bottle need to cool down to room temperature. For every new inoculation, the OD₇₅₀ is measured afterwards. Due to the experiences on microorganism cultures, a lower OD than 0.3 would lead to a higher risk of a cell mortalization.

The microorganisms were cultivated under certain stress conditions. Therefore, different phases of cultivation were performed, the growth phase (GP) and stationary phase (SP). While the microorganisms produced higher amounts of biomass in the GP, during the SP stress conditions were applied using modified conditions in the medium. GP cultivation is also denoted as Cultivation Method ID 1.

### Nitrogen starvation (Cultivation Method ID 2)

The N-starvation stress cultivation was achieved by executing a growth phase cultivation beforehand to reach an OD₇₅₀ of approximately 3.0 and exchanging the medium to 0N-BBM for the starvation process. After adaption of the medium to containing no nitrogen source the microorganisms grew by adapting their metabolism. Starting from an OD₇₅₀ of 3.0 the cultures were grown to reach at least 5.0, resulting in a sufficient amount of biomass for further testing.

### Salt stress (Cultivation Method ID 3)

Salt stress was induced by the use of 3N-BBM medium supplemented with 0.1 M Sodium Chloride. By increasing concentrations of salt in the nutrition medium, microorganisms growth was inhibited, resulting in small increase of the biomass in general. Starting from an OD₇₅₀ of 3.0 the cultures were grown to reach at least 4.0, indicating adaption to the media composition.

### Mixotrophic stress (Cultivation Method ID 4)

In this two-phase cultivation procedure, starting with the initial growth phase, a starvation of nitrogen and phosphate was performed. OD750 did increase substantially in the growth phase. Stress inductions were started with OD₇₅₀ of 5.0 to produce secondary metabolites in the stationary phase. Microorganism strains were cultivated using 3N-BBM medium until depletion of nitrogen and phosphate, subsequently continuing with ON-BBM medium without K₂HPO₄ and KH₂PO₄. The volume of media without nitrogen and phosphate was adjusted in the stress phase to generate higher cell densities. Therefore, media volume was reduced by half. Starting from an OD₇₅₀ of 3.0 the cultures were grown to reach at least 5.0 of cell density before applying the stress conditions.

### Glucose stress (Cultivation Method ID 5)

In the glucose stress factor cultivation, an amount of 5% glucose was added and fed again after 8 days. Here, growth increased rapidly due to the additional amount of nutrient, resulting in stress due to high cell densities in the reactor. Starting from an OD₇₅₀ of 3.0 the cultures were grown to reach at least 10.0, resulting in a sufficient amount of biomass.

After the cultivation the microorganism were harvested and the suspension filled in centrifuge bottles wherein the liquid phase could be separated and be discarded afterwards. Further, the microorganism pellets were washed with H₂O dest. as the final microorganism samples needed to be free of their respective cultivation medium. Therefore, the pellet was re-suspended with H₂O dest. and cleared for three times. After the third washing step the clean pellet was separated in weighted falcon tubes. Afterwards, the residues were lyophilized and stored at -20°C.

Biomasses obtained from the stress cultivation of nitrogen deprivation, increased amounts of salt and glucose, as well as a two-phase mixotrophic cultivation, were extracted and tested for their cytotoxicity and anti-inflammatory prosperities as follows.

### Extraction of microorganism biomass

For the extraction of bioactive compounds from soil microorganisms the centrifuged and washed microorganism biomass was lyophilized. To extract mostly polar compounds, the extraction solvents ethanol/water 8:2 (v/v) denoted as solvent 1 in tables and figures were used while mostly apolar compounds were extracted with dichloromethane/methanol 1:1 (v/v) denoted as solvent 2 in tables and figures. Ethanol/water 8:2 (v/v) has a dielectric constant of 35.7. Dichloromethane/methanol 1:1 (v/v) has a dielectric constant of 20.5.

100 mg microorganism biomass were transferred into a 5 ml Eppendorf cup together with 1 mg of glass beads (Ø 0.25-0.5 mm, Cat. No. A553.1, Carl Roth GmbH + Co. KG). After adding 4 ml of the respective solvent the mixture was vortexed for 3 min and centrifuged with maximum speed (11,000 rpm) for 15 min at RT. Supernatants were transferred into new vessels. The procedure was repeated 3 times. The collected supernatant was pooled and dried at RT o/n. Dry extracts were stored at - 20°C until further use.

Extracts were dissolved in 100 % DMSO to a final extract concentration of 10 mg/ml and filtrated sterile with DMSO resistant regenerated cellulose (RC) filters. DMSO stocks of extracts were protected from light and stored in 1 ml aliquots at -20°C (see M. Stranska-Zachariasova, P. Kastanek, Z. Dzuman, J. Rubert, M. Godula, and J. Hajslova, "Bioprospecting of microalgae: Proper extraction followed by high performance liquid chromatographic-high resolution mass spectrometric fingerprinting as key tools for successful metabolome characterization", Journal of Chromatography. B, Analytical Technologies in the Biomedical and Life Sciences, 1015-1016, pp. 22-33, 2016).

### Anti-inflammatory activity screening

### Inflammatory response by genotoxic stress or by cytokine induction

To identify extracts which are anti-inflammatory an inflammatory response screening can be performed in which UVB light induced genotoxic stress is applied in a NF-_{K}B reporter HaCaT cells. HaCaT is a spontaneously transformed aneuploid immortal keratinocyte cell line from adult human skin, widely used in scientific research (P. Boukamp, R. T. Petrussevska, D. Breitkreutz, J. Hornung, A. Markham, and N. E. Fusenig, "Normal keratinization in a spontaneously immortalized aneuploid human keratinocyte cell line", The Journal of Cell Biology, vol. 106, no. 3, pp. 761-771, 1988).

For generating the reporter cell line HaCaT NF-κB Luc cell line, the pHR NF-κB pTA Linker Luc pSV40-PAC WPPRE SIN-18 plasmid (Figure 1) was generated by inserting NF-κB enhancer element holding four κB binding sites (DNA consensus sequence 5'-GGGRNYYYCC-3') and a minimal promoter pTA before the firefly luciferase gene.

Additionally, a posttranscriptional regulatory element of the woodchuck hepatitis virus (WPRE) was implemented for the enhancement of gene expression. Flanking long terminal repeats (LTR) were utilized for genome integration. A puromycin resistance gene (PAC) transcriptionally regulated by the SV40 enhancer/promoter element was used for the selection of transduced cells. Infected cells which integrated the construct were subsequently selected with puromycin (Thermo Fisher Scientific A1113802).

In the assay the luciferase reporter gene expression under the control of NF-κB reporter elements was used as a readout for inflammatory response induced by UVB irradiation. Optimal density was achieved with 17,000 cells per well in a 96-well plate whereas cells should be confluent for treatment. The experimental set-up can be performed in two different ways upon irradiation with 0.15 J/cm² UVB light: post- and pre-treatment (D. Nothdurfter, "Identification of the anti-inflammatory potential of extracts from selected soil algae strains", Master's Thesis, University of Natural Resources and Applied Life Sciences, Vienna, 2018). In the post-treatment approach cells are treated after irradiation with extracts, while in the pre-treatment screening set-up extracts are applied on the cells 5 h before irradiation, to initiate the metabolite uptake by the cells.

For evaluation of the resulting data a cutoff of 5 % reduced reporter activity compared to the UVB-Control are regarded as a potential inhibitory effect. Treatment with extracts which show a reduced reporter activity in at least two independent experiments are considered as positive hits and were used for further verification.

For identification, extracts were given unique ID numbers to distinguish between microorganism strain, cultivation method, extraction solvents and replicate. The extract labeling nomenclature can be seen in Table 1.

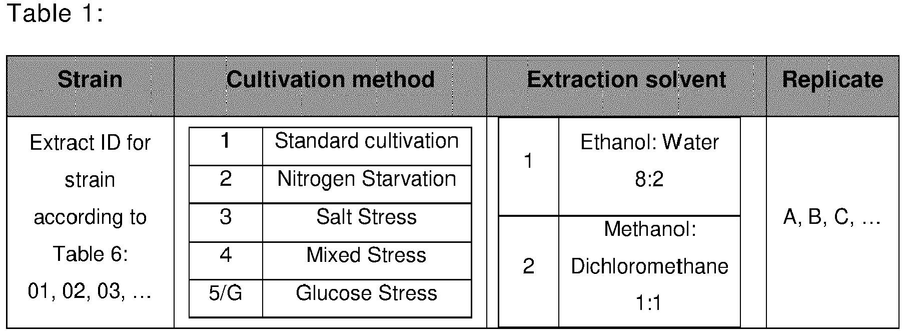

For extracts with "i" in the extract ID the microorganisms were cultivated shorter before harvest (half cultivation time).

The described UVB based inflammatory response reporter activity screening method was performed on several extracts and results are presented in Tables 2 and 3.

**Table 2:**

| | **UVB Pre Treatment** | | | **UVB Post Treatment** | | |
|---|---|---|---|---|---|---|
| **Extract ID** | **Extracts obtained with solvent 1** | | | | | |
| | **1st** | **2nd** | **3rd** | **1st** | **2nd** | **3rd** |
| **012B i** | 116% | 72% | 75% | 90% | 98% | 71% |
| **022C** | 81% | 69% | 98% | 84% | 88% | 84% |
| **182B** | 103% | 67% | 107% | 105% | 74% | 62% |
| **162B i** | 85% | 104% | 108% | 103% | 91% | 113% |
| **334B** | 100% | 87% | 92% | 96% | 104% | 103% |
| **012B** | 120% | 70% | 90% | 90% | 111% | 85% |
| **04G3** | 89% | 107% | 112% | 94% | 97% | 109% |
| **062C** | 93% | 102% | 112% | 89% | 90% | 81% |
| **062B i** | 86% | 92% | 103% | 78% | 94% | 112% |
| **212B i** | 89% | 95% | 103% | 115% | 112% | 101% |
| **104B** | 107% | 106% | 102% | 86% | 98% | 95% |
| **332B** | 91% | 100% | 95% | 71% | 124% | 91% |
| **112C** | 82% | 95% | 100% | 102% | 93% | 75% |
| **074B** | 93% | 102% | 96% | 102% | 80% | 77% |
| **04G2** | 118% | 84% | 104% | 91% | 86% | 95% |
| **274B** | 80% | 82% | 97% | 121% | 133% | 108% |
| **164B** | 95% | 107% | 97% | 97% | 66% | 132% |
| **204B** | 98% | 88% | 114% | 136% | 114% | 121% |
| **082B i** | 102% | 110% | 110% | 108% | 90% | 86% |
| **102B** | 112% | 104% | 92% | 71% | 95% | 116% |
| **352B** | 100% | 100% | 150% | 121% | 109% | 106% |
| **102C** | 122% | 112% | 86% | 90% | 118% | 86% |
| **174B** | 89% | 119% | 101% | 116% | 74% | 123% |
| **342B i** | 110% | 111% | 102% | 93% | 106% | 138% |
| **332C** | 105% | 134% | 99% | 93% | 101% | 108% |
| **092B i** | 92% | 113% | 92% | 135% | 103% | 99% |
| **342B** | 114% | 132% | 95% | 92% | 106% | 114% |
| **264B** | 72% | 93% | 99% | 134% | 122% | 124% |
| **162C** | 84% | 111% | 111% | 126% | 144% | 97% |
| **262B** | 66% | 72% | 109% | 130% | 121% | 136% |
| **094B** | 113% | 101% | 104% | 118% | 113% | 117% |
| **102B i** | 94% | 109% | 113% | 75% | 103% | 108% |
| **332B i** | 89% | 100% | 108% | 115% | 138% | 105% |
| **214B** | 101% | 109% | 104% | 128% | 138% | 105% |
| **092C1** | 138% | 102% | 93% | 135% | 131% | 86% |
| **082B** | 114% | 118% | 115% | 131% | 83% | 80% |
| **04G4** | 110% | 120% | 103% | 73% | 111% | 101% |
| **184B** | 108% | 107% | 102% | 120% | 139% | 107% |
| **182B i** | 113% | 111% | 92% | 134% | 138% | 103% |
| **092C2** | 146% | 100% | 109% | 138% | 140% | 90% |
| **04G1** | 100% | 107% | 117% | 130% | 147% | 106% |
| **084B** | 127% | 109% | 100% | 158% | 112% | 92% |
| **262B i** | 99% | 126% | 127% | 113% | 130% | 136% |
| **202C** | 143% | 84% | 123% | 170% | 135% | 113% |
| **092B** | 109% | 135% | 146% | 139% | 125% | 131% |
| **252C** | 106% | 134% | 114% | 144% | 114% | 170% |

**Table 3:**

| | **UVB Pre Treatment** | | | **UVB Post Treatment** | | |
|---|---|---|---|---|---|---|
| **Extract ID** | **Extracts obtained with solvent 2** | | | | | |
| | **1st** | **2nd** | **3rd** | **1st** | **2nd** | **3rd** |
| **012B i** | 83% | 96% | 84% | 87% | 75% | 100% |
| **022C** | 92% | 108% | 91% | 94% | 72% | 102% |
| **182B** | 71% | 94% | 110% | 92% | 98% | 91% |
| **162B i** | 67% | 89% | 99% | 77% | 86% | 70% |
| **334B** | 57% | 89% | 107% | 93% | 80% | 93% |
| **012B** | 75% | 99% | 84% | 121% | 78% | 90% |
| **04G3** | 85% | 80% | 93% | 106% | 92% | 81% |
| **062C** | 89% | 94% | 95% | 99% | 108% | 95% |
| **062B i** | 78% | 96% | 103% | 110% | 111% | 93% |
| **212B i** | 87% | 119% | 76% | 91% | 94% | 82% |
| **104B** | 85% | 97% | 73% | 110% | 103% | 106% |
| **332B** | 91% | 109% | 92% | 118% | 108% | 78% |
| **112C** | 96% | 111% | 76% | 112% | 112% | 114% |
| **074B** | 86% | 101% | 99% | 132% | 111% | 91% |
| **04G2** | 108% | 102% | 82% | 116% | 87% | 99% |
| **274B** | 82% | 85% | 104% | 84% | 118% | 84% |
| **164B** | 122% | 73% | 86% | 97% | 103% | 104% |
| **204B** | 83% | 88% | 68% | 97% | 92% | 93% |
| **082B i** | 79% | 94% | 83% | 140% | 93% | 102% |
| **102B** | 106% | 100% | 94% | 103% | 95% | 110% |
| **352B** | 67% | 96% | 67% | 83% | 102% | 105% |
| **102C** | 102% | 77% | 98% | 126% | 94% | 100% |
| **174B** | 134% | 71% | 78% | 91% | 104% | 114% |
| **342B i** | 57% | 94% | 103% | 108% | 104% | 94% |
| **332C** | 73% | 96% | 98% | 96% | 105% | 113% |
| **092B i** | 96% | 102% | 92% | 126% | 94% | 81% |
| **342B** | 86% | 121% | 63% | 110% | 104% | 95% |
| **264B** | 99% | 85% | 106% | 100% | 117% | 83% |
| **162C** | 94% | 93% | 80% | 101% | 104% | 90% |
| **262B** | 94% | 94% | 126% | 98% | 95% | 103% |
| **094B** | 87% | 95% | 93% | 114% | 96% | 96% |
| **102B i** | 94% | 112% | 90% | 121% | 117% | 115% |
| **332B i** | 98% | 98% | 83% | 106% | 116% | 96% |
| **214B** | 100% | 96% | 87% | 95% | 96% | 98% |
| **092C1** | 75% | 86% | 109% | 127% | 102% | 87% |
| **082B** | 101% | 109% | 114% | 128% | 86% | 107% |
| **04G4** | 89% | 99% | 155% | 134% | 97% | 101% |
| **184B** | 113% | 97% | 88% | 101% | 104% | 133% |
| **182B i** | 135% | 74% | 91% | 106% | 110% | 120% |
| **092C2** | 91% | 90% | 98% | 120% | 107% | 108% |
| **04G1** | 93% | 83% | 94% | 143% | 101% | 124% |
| **084B** | 99% | 107% | 115% | 122% | 102% | 114% |
| **262B i** | 105% | 99% | 172% | 94% | 94% | 104% |
| **202C** | 87% | 74% | 110% | 151% | 123% | 96% |
| **092B** | 101% | 120% | 82% | 124% | 128% | 87% |
| **252C** | 117% | 125% | 101% | 91% | 97% | 117% |

Selected hits in the screening test have been further confirmed by Cohen's d test (Cohen, J. (1988), Statistical Power Analysis for the Behavioral Sciences (2nd ed), Hillsdale, N.J: L. Erlbaum Associates.). This test is used to estimate the effect size of the extract treatment depending on mean values of the measured luminescence signals. Extracts can be seen as hits when resulting in a Cohen's d of 1.0 or higher. Cohen's d test values of genotoxic stress screening are presented in Table 4.

**Table 4:**

| Extracts obtained with solvent 1 | | | Extracts obtained with solvent 2 | | |
|---|---|---|---|---|---|
| Extract ID | Cohen's d | | Extract ID | Cohen's d | |
| | Pre-Treatment | Post-Treatment | | Treatment | Post-Treatment |
| 0421C | 0.6 | 0.7 | 0422C | 2.3 | 2.9 |
| 0451C | 1.7 | 1.8 | 0452C | 1.6 | 1.8 |
| 0521C | 0.5 | 0.6 | 0522C | 1.6 | 0.4 |
| 0821B | 2.8 | 2.7 | 0822B | 0.7 | 2.0 |
| 1721C | 0.7 | 2.3 | 1722C | 1.2 | 1.1 |
| 1741B | 1.4 | 0.6 | 1742B | 2.2 | 2.0 |
| 1921C | 1.9 | 1.8 | 1922C | 1.4 | 1.2 |
| 2021C | 1.3 | 1.8 | 2022C | 0.8 | 0.6 |
| 2321C | 0.8 | 0.6 | 2322C | 0.8 | 1.2 |
| 2521C | 1.8 | 2.1 | 2522C | 0.7 | 0.9 |
| 2811C | 1.4 | 1.1 | 2812C | 2.1 | 0.8 |
| 3221B | 1.2 | 2.3 | 3222B | 0.6 | 0.8 |
| 3321B | 1.4 | 1.7 | 3322B | 0.5 | 0.6 |

Furthermore, for determining the inflammatory response induced by the cytokine TNFα NF-κB reporter HaCaT cell were treated with 0.75 ng/ml TNFα in the medium and incubated for 6 h. Again, selected hits in the screening test were further confirmed by Cohen's d test. The TNFα based inflammatory response reporter activity screening method was performed on several extracts and results are presented in Table 5. Respective Cohen's d test values of cytokine induction screening are presented in Table 6.

**Table 5:**

| | **TNFα treatment** | | | | | |
|---|---|---|---|---|---|---|
| **Extract ID** | **Extracts obtained with solvent 1** | | | **Extracts obtained with solvent 2** | | |
| | **1st** | **2nd** | **3rd** | **1st** | **2nd** | **3rd** |
| **012B i** | 95% | 86% | 91% | 89% | 85% | 105% |
| **012B** | 96% | 91% | 92% | 114% | 88% | 77% |
| **022C** | 85% | 75% | 84% | 68% | 85% | 64% |
| **04G1** | 89% | 87% | 97% | 128% | 88% | 89% |
| **04G2** | 96% | 89% | 102% | 92% | 80% | 90% |
| **04G3** | 100% | 100% | 93% | 98% | 82% | 79% |
| **04G4** | 86% | 103% | 81% | 72% | 85% | 61% |
| **062B i** | 93% | 88% | 74% | 70% | 104% | 62% |
| **062C** | 111% | 81% | 80% | 97% | 109% | 73% |
| **074B** | 96% | 73% | 73% | 87% | 101% | 82% |
| **082B i** | 86% | 87% | 93% | 87% | 95% | 76% |
| **082B** | 87% | 77% | 73% | 120% | 91% | 74% |
| **084B** | 84% | 69% | 76% | 86% | 71% | 86% |
| **092B i** | 91% | 83% | 84% | 87% | 99% | 90% |
| **092C1** | 81% | 99% | 80% | 96% | 74% | 92% |
| **092C2** | 91% | 87% | 101% | 109% | 93% | 84% |
| **092B** | 82% | 82% | 81% | 73% | 66% | 72% |
| **094B** | 82% | 104% | 96% | 63% | 65% | 90% |
| **102B i** | 85% | 91% | 86% | 85% | 89% | 74% |
| **102B** | 89% | 86% | 84% | 105% | 80% | 84% |
| **102C** | 75% | 76% | 86% | 78% | 92% | 54% |
| **104B** | 82% | 74% | 88% | 101% | 103% | 85% |
| **112C** | 79% | 78% | 85% | 93% | 103% | 94% |
| **162B i** | 75% | 82% | 83% | 73% | 97% | 71% |
| **162C** | 82% | 87% | 82% | 113% | 108% | 105% |
| **164B** | 86% | 99% | 76% | 90% | 79% | 69% |
| **174B** | 102% | 89% | 112% | 114% | 117% | 111% |
| **182B i** | 108% | 89% | 94% | 85% | 96% | 91% |
| **182B** | 99% | 87% | 112% | 81% | 77% | 93% |
| **184B** | 108% | 88% | 98% | 97% | 96% | 97% |
| **202C** | 94% | 90% | 109% | 57% | 89% | 61% |
| **212B i** | 85% | 108% | 98% | 68% | 105% | 80% |
| **214B** | 85% | 106% | 97% | 84% | 100% | 87% |
| **252C** | 77% | 92% | 97% | 91% | 102% | 110% |
| **262B i** | 90% | 93% | 109% | 101% | 78% | 84% |
| **262B** | 82% | 91% | 84% | 73% | 88% | 85% |
| **264B** | 96% | 85% | 80% | 73% | 76% | 92% |
| **274B** | 103% | 77% | 74% | 78% | 87% | 92% |
| **332B i** | 99% | 84% | 90% | 99% | 97% | 86% |
| **332B** | 104% | 84% | 69% | 75% | 107% | 113% |
| **332C** | 92% | 82% | 69% | 87% | 108% | 102% |
| **334B** | 86% | 97% | 65% | 86% | 118% | 90% |
| **342B i** | 65% | 87% | 66% | 82% | 77% | 97% |
| **342B** | 77% | 97% | 62% | 77% | 104% | 89% |
| **352B** | 82% | 92% | 64% | 85% | 83% | 83% |

**Table 6:**

| Extracts obtained with solvent 1 | | Extracts obtained with solvent 2 | |
|---|---|---|---|
| Extract ID | Cohen's d | Extract ID | Cohen's d |
| 0221C | 2.5 | 0222C | 3.5 |
| 04G21 | 0.7 | 04G22 | 2.3 |
| 0621B i | 2.0 | 0622B i | 1.8 |
| 0841B | 2.7 | 0842 B | 1.7 |
| 0921B | 2.3 | 0922B | 3.1 |
| 0941B | 2.1 | 0942 B | 3.1 |
| 1021C | 2.4 | 1022C | 1.7 |
| 1041B | 2.0 | 1042B | 0.9 |
| 1621C | 2.4 | 1622C | 0.7 |
| 1621B i | 2.4 | 1622B i | 2.0 |
| 1641B | 1.6 | 1642B | 2.6 |
| 2021C | 1.1 | 2022C | 3.8 |
| 2041B | 1.6 | 2042 B | 2.5 |
| 3421B i | 1.8 | 3422B i | 1.4 |
| 3521B | 1.7 | 3522B | 2.2 |

In summary, the above anti-inflammatory activity screenings revealed that extracts from the algae and Cyanobacteria presented in Table 7 are anti-inflammatory.

**Table 7:**

| **Extract ID** | **Straiin ID** | **Collection ID** | **Characterization** |
|---|---|---|---|
| **02** | MCI-36 | BS110 | *Coccomyxa sp.:* |
| | | | *Phylum Chlorophyta* |
| | | | *Subphylum Chlorophytina* |
| | | | *Class Trebouxiophyceae* |
| | | | *Order Trebouxiophyceae* |
| | | | *Family Coccomvxaceae* |
| **04** | MCI-31 | V46 | *Chromochloris zofingiensis:* |
| | | | *Phylum Chlorophyta* |
| | | | *Subphylum Chlorophytina* |
| | | | *Class Chlorophyceae* |
| | | | *Order Sphaeropleales* |
| | | | *Family Chromochloridaceae* |
| | | | *Genus Chromochloris* |
| **05** | MCI-22 | V168 | *Xanthophyceae* |
| **08** | MCI-8 | BS775 | *Pseudochlorella subsphaerica:* |
| | | | *Phylum Chlorophyta* |
| | | | *Subphylum Chlorophytina* |
| | | | *Class Trebouxiophyceae* |
| | | | *Order Prasiolales* |
| | | | *Family Koliellaceae* |
| | | | *Genus Pseudochlorella* |
| **09** | MCI-12 | IB273 (PUFA) | *Coccomyxa sp:* |
| | | | *Phylum Chlorophyta* |
| | | | *Subphylum Chlorophytina* |
| | | | *Class Trebouxiophyceae* |
| | | | *Order Trebouxiophyceae* |
| | | | *Family Coccomvxaceae* |
| **10** | MCI-13 | IB410 | *Neocystis brevis:* |
| | | | *Phylum Chlorophyta* |
| | | | *Subphylum Chlorophytina* |
| | | | *Class Chlorophyceae* |
| | | | *Order Sphaeropleales* |
| | | | *Family Radiococcaceae* |
| | | | *Genus Neocystis* |
| **16** | MCI-47 | IB408 | *Chromochloris zofingiensis:* |
| | | | *Phylum Chlorophyta* |
| | | | *Subphylum Chlorophytina* |
| | | | *Class Chlorophyceae* |
| | | | *Order Sphaeropleales* |
| | | | *Family Chromochloridaceae* |
| | | | *Genus Chromochloris* |
| **17** | MCI-34 | CV. Vulgaris | *Chlorella saccharophila:* |
| | | | *Phylum Chlorophyta* |
| | | | *Subphylum Chlorophytina* |
| | | | *Class Trebouxiophyceae* |
| | | | *Order Chlorellales* |
| | | | *Family Chlorellaceae* |
| | | | *Genus Chlorella* |
| **19** | MCI-23 | V195 | *Bracteacoccus sp.:* |
| | | | *Phylum Chlorophyta* |
| | | | *Subphylum Chlorophytina* |
| | | | *Class Chlorophyceae* |
| | | | *Order Sphaeropleales* |
| | | | *Family Bracteacoccaceae* |
| | | | *Genus Bracteacoccus* |
| **20** | MCI-21 | V142 | *Chromochloris zofingiensis:* |
| | | | *Phylum Chlorophyta* |
| | | | *Subphylum Chlorophytina* |
| | | | *Class Chlorophyceae* |
| | | | *Order Sphaeropleales* |
| | | | *Family Chromochloridaceae* |
| | | | *Genus Chromochloris* |
| **23** | MCI-49 | T87 | *Bracteacoccus sp.:* |
| | | | *Phylum Chlorophyta* |
| | | | *Subphylum Chlorophytina* |
| | | | *Class Chlorophyceae* |
| | | | *Order Sphaeropleales* |
| | | | *Family Bracteacoccaceae* |
| | | | *Genus Bracteacoccus* |
| **25** | MCI-25 | V204 | *Dictyococcus varians:* |
| | | | *Phylum Chlorophyta* |
| | | | *Subphylum Chlorophytina* |
| | | | *Class Chlorophyceae* |
| | | | *Order Sphaeropleales* |
| | | | *Family Dictyococcaceae* |
| | | | *Genus Dictyococcus* |
| **28** | MCI-7 | BS319 | *Muriella sp.:* |
| | | | *Phylum Chlorophyta* |
| | | | *Subphylum Chlorophytina* |
| | | | *Class Trebouxiophyceae* |
| | | | *Order Chlorellales* |
| | | | *Family Chlorellaceae* |
| **33** | MCI-35 | CH. | *Coelastrella sp.:* |
| | | | *Phylum Chlorophyta* |
| | | | *Subphylum Chlorophytina* |
| | | | *Class Chlorophyceae* |
| | | | *Order Sphaeropleales* |
| | | | *Family Scenedesmaceae* |
| **34** | MCI-53 | BS363 | *Nostoc sp.:* |
| | | | *Phylum Cyanobacteria* |
| | | | *Class Cyanophyceae* |
| | | | *Subclass Nostocophycidae* |
| | | | *Order Nostocales* |
| | | | *Family Nostocaceae* |
| | | | *Genus Nostoc* |
| **35** | MCI-55 | V208 | *Bracteacoccus sp.:* |
| | | | *Phylum Chlorophyta* |
| | | | *Subphylum Chlorophytina* |
| | | | *Class Chlorophyceae* |
| | | | *Order Sphaeropleales* |
| | | | *Family Bracteacoccaceae* |
| | | | *Genus Bracteacoccus* |

Strain ID was determined by the Management Center Innsbruck, Department of Biotechnology. Collection ID was determined by the botanical institute of the University of Innsbruck (see G. Gaertner, "ASIB - The culture collection of algae at the Botanical Institute of the University at Innsbruck (Austria) Catalogue of Strains 1996": in Berichte des naturwissenschaftlichen-medizinischen Verein, Band 83, 1996, pp. 45-69).

### Dose dependency of anti-inflammatory effect of extracts

Extracts were further investigated by testing the effect in a concentration dependent manner. For the validation of the hits, 0.75 µg/ml TNFα has been used to induce the inflammatory response.

The extract concentrations used for the dose-dependent validation were 50 µg/ml , 10 µg/ml , 1 µg/ml , 0.1 µg/ml and 0.01 µg/ml resulting in a maximum DMSO concentration of 0.5 % during treatment of cells.

Extracts with dose-dependent effects and measurable average decrease of luciferase signal below 90 % compared to the control at a concentration of 10 µg/ml were selected as validated hits.

Seven extracts fulfilled these criteria, which were 0221C, 0841B, 3421B i, 3421B, 0922B and 2022C. The data showed that treatment with extracts 3421B, 3421B i and 2022C resulted in the most pronounced dose dependent anti-inflammatory effects.

The data thus showed a pronounced dose-dependent effect after treatment of cells with the extracts of *Coccomyxa sp., Pseudochlorella subsphaerica, Nostoc sp. and Chromochloris zofingiensis.*

The extracts of *Coccomyxa sp.* show an average decrease in reporter activity of 4% at 10 µg/ml and 14% at 50 µg/ml extract concentration was detected.

The extracts of *Pseudochlorella subsphaeric* show an average decrease in reporter activity of 17% at 10 µg/ml and 26% at 50 µg/ml extract concentration was detected.

The extracts of *Nostoc sp.* show an average decrease in reporter activity of 13% at 10 µg/ml and 33% at 50 µg/ml extract concentration was detected. The extracts of *Chromochloris zofingiensis* show an average decrease in reporter activity of 32% at 10 µg/ml and 75% at 50 µg/ml extract concentration was detected.

### Assessment of cytotoxicity of extracts

To check for possible cytotoxicity of the extracts in further experiments, an evaluation of toxic effects on the viability of HaCaT cells was performed. For this purpose, a resazurin assay has been used to test different concentrations of extracts, ranging from high concentrations (100 µg/ml, 1 % DMSO) to low concentrations (0.1 µg/ml, 0.1 % DMSO).

Resazurin is a non-fluorescent blue dye reduced by metabolic active cells to the fluorescent Resorufin which can be quantified by fluorescence measurements (Ext. 560 nm, Emi. 590 nm) (R. S. TWIGG, "Oxidation-Reduction Aspects of Resazurin," Nature, vol. 155, no. 3935, pp. 401-402, 1945). Cytotoxic substances lower the activity of cells, which leads to the slowing down of Resazurin turnover, causing a reduction of fluorescent signal, which is proportional to the metabolic activity of the culture (J. O'Brien, I. Wilson, T. Orton, and F. Pognan, "Investigation of the Alamar Blue (resazurin) fluorescent dye for the assessment of mammalian cell cytotoxicity," European Journal of Biochemistry, vol. 267, no. 17, pp. 5421-5426, 2000). The supernatant was aspirated and cells were washed with pre-warmed DPBS. 100 µl medium with 2 % FBS and 10 % resazurin solution (working stock concentration 0.15 mg/ml in DPBS) were added and incubated at 37°C, 5 % CO2 and 90 % humidity for 3 h (confluent cells) or 4 h (proliferative cells). Fluorescence signal was measured with a plate reader at excitation/emission wavelength of 540 nm/590 nm.

Every extract was prepared as a stock of 10 mg/ml in 100 % DMSO for further testing. For evaluation of cytotoxicity in HaCaT cells we added the stock to the final concentrations of 100 µg/ml , 10 µg/ml, 1 µg/ml and 0.1 µg/ml in medium. The extracts with a concentration of 100 µg/ml resulted in a presence of 1 % DMSO, whereas the other dilutions caused 0.1 % DMSO in the medium. Those concentrations of DMSO were included as toxicity controls in cultures.

The described toxicity test was performed on several extracts and results are shown in Table 8:

**Table 8: Toxicity Assay Screening of extracts: Results normalized to control cells.**

| **Extract ID** | **Toxicity Assay Screening** | | | | |
|---|---|---|---|---|---|
| | **10 µg/ml** | **10 µg/ml** | **1 µg/ml** | **0.1 µg/ml** | **Ctr w/o cells** |
| **0221C** | 92% | 100% | 101% | 99% | 101% |
| **0222C** | 112% | 108% | 111% | 108% | 104% |
| **0421C** | 91% | 101% | 101% | 99% | 101% |
| **0422C** | 91% | 96% | 107% | 102% | 101 % |
| **04G21** | 92% | 106% | 106% | 105% | 106% |
| **04G22** | 102% | 104% | 105% | 105% | 104% |
| **0521C** | 92% | 99% | 99% | 101% | 99% |
| **0522C** | 90% | 102% | 108% | 103% | 103% |
| **0841B** | 78% | 105% | 103% | 105% | 107% |
| **0842B** | 92% | 103% | 108% | 101% | 123% |
| **0921B** | 99% | 106% | 109% | 103% | 105% |
| **0922B** | 86% | 103% | 103% | 100% | 113% |
| **0941B** | 86% | 105% | 108% | 106% | 101% |
| **0942B** | 91% | 96% | 101% | 92% | 105% |
| **1021C** | 69% | 99% | 102% | 101% | 108% |
| **1022C** | 93% | 98% | 102% | 100% | 103% |
| **1621C** | 59% | 105% | 103% | 103% | 113% |
| **1622C** | 103% | 108% | 109% | 106% | 105% |
| **1721C** | 95% | 102% | 104% | 103% | 101% |
| **1722C** | 102% | 108% | 108% | 105% | 111% |
| **1741B** | 102% | 103% | 104% | 107% | 111% |
| **1742B** | 91% | 97% | 97% | 96% | 102% |
| **1921C** | 92% | 101% | 101% | 101% | 101% |
| **1922C** | 96% | 100% | 101% | 100% | 105% |
| **2021C** | 91% | 100% | 101% | 100% | 99% |
| **2022C** | 99% | 103% | 101% | 100% | 108% |
| **2321C** | 89% | 98% | 100% | 99% | 100% |
| **2322C** | 92% | 101% | 103% | 102% | 103% |
| **2521C** | 94% | 103% | 107% | 106% | 106% |
| **2522C** | 95% | 102% | 99% | 101% | 102% |
| **2821C** | 96% | 102% | 102% | 100% | 104% |
| **2822C** | 89% | 99% | 100% | 102% | 103% |
| **3321Bi** | 100% | 102% | 106% | 105% | 98% |
| **3322Bi** | 97% | 99% | 103% | 102% | 97% |
| **3421C** | 97% | 105% | 103% | 103% | 104% |
| **3422C** | 95% | 99% | 99% | 100% | 101% |
| **3521B** | 56% | 109% | 108% | 106% | 106% |
| **3522B** | 86% | 99% | 100% | 102% | 103% |

Specifically, all extracts of algae and cyanobacteria listed in table 7 revealed that concentrations 10 to 0.1 µg/ml result in a low decrease and did not affect the viability significantly.

### Cytokine release in Reconstituted Human Epidermis model after treatment with extracts

The bioactivity of the extract of *Chromochloris zofingiensis* (2022C) was tested in human epidermis equivalents by measuring cytokine secretion in response to TNFα treatment.

The extract was tested in a physiological relevant set-up represented by reconstructed human epidermis (RHE)-models (Figure 2), (W. Pixelproduction, epiCS - CellSystems GmbH. [Online]. Available: https://cellsystems.de/cells-and-media/c/epics (accessed: Oct. 2 2020)), purchased from Cell Systems. epiCS RHE models are validated for the classification of compounds according to the OECD test guideline 431. RHE are generated from primary human keratinocytes and form a multilayered, highly differentiated model of the human epidermis that mimics biochemical as well as physiological properties of the upper skin. The cellular structure of RHE closely resembles the human epidermis including the basal layer (stratum basale), the spinous layer (stratum spinosum), the granular layer (stratum granulosum) and the outermost cornified layer (stratum corneum) with intact barrier function. The RHE models were grown at the air-liquid interphase and therefore could be used for topical application and application via the medium.

The secretion of relevant pro-inflammatory cytokines of RHE-models (from Cell Systems Biotechnology), were quantified after induction with 0.75 ng/ml TNFα and treatment with DMSO, WFA or the extract of *Chromochloris zofingiensis* in a concentration of 10 µg/ml in DMSO.

After culturing the keratinocyte cell layer in cell culture inserts (0.6 cm²) under submerged conditions, the tissues were lifted to the air-liquid interphase to induce differentiation, epithelial stratification and cornification. The cellular structure of RHE-models resembles fully differentiated natural epidermis showing a base membrane, proliferating keratinocytes and a stratum corneum with an intact barrier function (Figure 2). Extracts can be either applied through the medium from underneath (mimicking the blood stream supply) or from above at the liquid/air interface (mimicking topical skin application). To test whether the extract of *Chromochloris zofingiensis* inhibits the release of cytokines in response to TNFα the RHE models were treated by adding the extract into the cultivating medium. The cytokine release was evaluated 24h and 48h after the TNFα treatment. The secretion of the cytokines IL-1ra, RANTES, IL-1α, GM-CSF, TSLP, IP-10, IL-18, IL-6, IL-8, IL-10, MCP-1, IL-1β, TNFα and INF-β was measured.

The experiments were performed in technical duplicates, which were represented by data points in close proximity of each other and repeated with 3 batches of epiCS to gain independent biological triplicates. The measurement and altitude of cytokine release was influenced by various conditions (time, temperature, distribution on the surface/in the medium of samples).

When equivalents where treated with DMSO (Ctr) no increase in cytokine release was detected. After treatment with TNFα and DMSO a pronounced increase of IL-10, MCP-1, IP-10 and RANTES (Figure 3) was observed. When cells were treated with the control inhibitor WFA a substantial decrease in release of the mentioned cytokines was detected. When the extract of *Chromochloris zofingiensis* was applied, an extended inhibition in cytokine release after 24 and 48 h, was observed, suggesting an anti-inflammatory effect of the extract. In particular, it was observed that the extract inhibited IL-10 and IP-10 and to a lower extend MCP-1 and had only moderate effect on the release of RANTES.

In summary, the results showed that the extract of *Chromochloris zofingiensis* reduced the release of pro-inflammatory cytokines, which indicated that components in this extract have anti-inflammatory effects in a skin set-up.

### Direct inhibition of IKKβ Kinase by the extracts in an in vitro kinase assay

The IKK complex subunit IKKβ is responsible for the phosphorylation of IkBα and it's targeting for degradation upon TNFα induction. Therefore, it has been investigated whether the extracts inhibit IKKβ. To demonstrate that compounds present in the extracts inhibit the relevant kinase, an *in vitro* immunoprecipitation kinase assay has been performed. The kinase assay was based on quantifying the activity of the kinase IKKβ via autoradiography.

Specifically, HEK 293T cells were transfected with the HA-IKKβ construct. Cells were washed with ice-cold phosphate-buffered saline (PBS) and extracted with lysis buffer containing 50 mM HEPES (pH 7.5), 150 mM NaCl, 1 % (vol/vol) Nonidet P-40, 2 mM EDTA, 50 mM sodium fluoride, 10 mM sodium pyrophosphate, 1 mM sodium orthovanadate, 50 µg of leupeptin (Boehringer Mannheim)/ml, 0.5 % aprotinin (Sigma), 1 mM phenylmethylsulphonyl fluoride (Sigma), and 3 mM benzamidine (Sigma). 300 ng of total protein were immunoprecipitated with 2 µg of anti-HA antibodies and immune complexes were collected using protein A-Sepharose beads (Amersham Pharmacia Biotech). Immune complexes were washed three times with lysis buffer followed by a single wash with high salt buffer (500 mM NaCl) and kinase assay buffer (50 mM HEPES [pH 7.5], 10 mM MgCl2, 1 mM dithiothreitol, 10 mM β-glycerophosphate). The kinase reaction was initiated by resuspending the beads in 25 µl of kinase assay buffer supplemented with 1 mM ATP, 8 µCi of [γ-p32] ATP (Amersham Pharmacia Biotech), and varying amounts of the extract of *Chromochloris zofingiensis* (0.4, 0.7, 1.4 µl) or the same volumes of DMSO. Recombinant His-IκBα was added in the amount 2.5 µg as a substrate. The reaction was carried out at 30°C for 45 min and terminated by the addition of sodium dodecyl sulphate (SDS)-polyacrylamide gel electrophoresis (PAGE) sample buffer and boiling the mixture for 5 min. Samples were subjected to 10 % SDS-PAGE, and the amount of ³²P incorporated into the IKKβ and IkBα proteins was assessed by autoradiography (Figure 4).

By looking at the substrate IkBα protein, a shift can be seen in the SDS-PAGE staining with Coomassie, suggesting it's phosphorylation. Indeed, upon examining the radioactive signaling, it can be seen that it was phosphorylated. Different concentrations of DMSO didn't interfere with the phosphorylation of the IkBα protein, while the extract of *Chromochloris zofingiensis* inhibited IκBα phosphorylation in a concentration dependent manner. Furthermore, the extract of *Chromochloris zofingiensis* hindered IKKβ to phosphorylate its specific substrate, the IkBα protein. However, it was also possible that components of the extract interfered with the interaction and recognition of the substrate of the kinase. Because the kinase in this assay can autophosphorylate itself we looked also at the autophosphorylation events and found that the extract 2022C of *Chromochloris zofingiensis* not only inhibited phosphorylation of the substrate but also autophosphorylation of the kinase, suggesting that it exerted a direct effect on the kinase activity. It can be concluded that the extract of *Chromochloris zofingiensis* indeed inhibited intrinsic kinase activity. The inhibition of IkBα degradation and phosphorylation by the extract in cells was caused by the direct inhibition of the IKK kinase activity by components in the extract of *Chromochloris zofingiensis* extract.

### Bioactivity guided fractionation and investigation of active metabolite using High performance liquid chromatography mass spectrometry

To identify the active metabolites in the extract 2022C of *Chromochloris zofingiensis* a bioactivity guided fractionation was performed using the Waters Acquity Polymer Chromatography System with a C18 column suited for preparative chromatography. Extracted components were separated based on differences in their physicochemical properties and screened again for their biological activity using the inflammatory response assay in reporter HaCaT NF-κB cells clone 4.

For development of analytical separation and identification of high-value metabolites a versatile and reproducible ultra-performance liquid chromatography (UPLC)-based separation system (Thermo Scientific), coupled to a high-resolution mass spectrometer (Bruker) operating in MS as well as all-ion fragmentation mode, was used.

Fractionation of extracts was done by size-exclusion chromatography on Kinetex 5µm C18 100A 250x10 mm column. Used HPLC system was Waters Aquity Arc System with quaternary solvent manager, thermostat, sample manager, fraction manager und isocratic solvent manager as prepump to MS. Solvents for HPLC separation were 0.1 % formic acid in water (solvent A) and Acetonitrile (solvent B). The gradient profile was as follows: 24 min from 10 % B to 90 % B, 30 min 10 % B. Flow was 3 ml/min with an injection volume of 0.5 ml. Measuring range 100-800 m/z with Waters QDA single quad mass spectrometer in Electrospray positive and negative alternating mode. PDA detector: ACQUITY Arc 2998 PDA Detector 200-800nm.

The active fraction which showed inhibition with NF-κB activation were analyzed by a quadrupole mass spectrometer (Bruker) coupled to a uHPLC-qTOF system (Thermo Fisher Scientific). Solvents for HPLC separation were 0.1 % formic acid in water (solvent A) and Acetonitrile (solvent B). The gradient profile was as follows: 16 min from 50 % to 90 % B; 2 min from 90 % to 90 % B. Flow was 0.5 ml/min with an injection volume of 10 µl. The analyses were performed in a data-dependent acquisition mode using a top 15 collision-induced dissociation method at 60,000 resolution.

The Q Exactive Plus mass spectrometer was operating in the data-dependent mode selecting the top 12 most abundant isotope patterns with charge >1 from the survey scan with an isolation window of 1.6 mass-to-charge ratio (m/z). Survey full scan MS spectra were acquired from 300 to 1750 m/z at a resolution of 70,000 with a maximum injection time of 120 ms, and automatic gain control target 1x106. The selected isotope patterns were fragmented by higher-energy collisional dissociation with normalized collision energy of 28 at a resolution of 35,000 with a maximum IT of 120 ms, and AGC target 5x105.

For the qualitative analysis of betaine lipids, an internal stable-isotope standard (Avanti Polar Lipids, Inc.) DGTS, with a concentration of 7 mM stock concentration in DMSO, was used. After calibration with the ionization efficiency at a series proportion of the mobile phase, the content of DGTS was identified by semi-quantitative analysis using the area ratio of each composition to the corresponding internal stable-isotope standard.

First, the analytical procedures to separate the components for further identification in a uHPLC-qTOF-MS/MS system were tested. This optimized run was transferred to preparative experiments in a large scale to separate fractions for further testing. Solvents for the HPLC separation were 0.1 % Formic acid in water (solvent A) and Acetonitrile (solvent B). Using the optimized separation run of 30 min with a gradient profile from 0 to 24 min, 10 % B to 90 % B, 30 min 10 % B, samples were collected every 0.5 min after 10 min runtime, resulting in 20 individual fractions (Figure 5) (see M. G. Weller, "A unifying review of bioassay-guided fractionation, effect-directed analysis and related techniques," Sensors (Basel, Switzerland), vol. 12, no. 7, pp. 9181-9209, 2012). Fractions were dried and redissolved in DMSO to obtain concentrations of 1 mg/ml for further testing in the inflammatory response assay.

In the inflammatory response assay HaCaT NF-κB cells were treated with 0.75 ng/ml TNFα and the individual fractions with a final concentration of 1 µg/ml for 6 h. Analysis of the inflammatory response (Figure 6) showed a decrease of reporter signal in fractions 7 and 8 which corresponds with the peak at runtime 17.05 min detected in the ESI positive mode and 17.06 min in the negative mode.

Fractions 7 and 8 resulted in the most pronounced anti-inflammatory response and were further analyzed with the uHPLC-qTOF-MS/MS system. These samples were combined, because the detected signals in the mass spectrometry overlapped due to the same metabolites in the tested samples.

The pooled samples were further named fraction 8. In the analysis of fraction 8 one peak was predominantly detected in the positive mode. Further investigation showed a fragmentation pattern which corresponded to the molecule DGTS (diacylglyceryl-*N,N,N*-trimethylhomoserine) containing two C16:0 fatty acids (Figure 7). The fragmentation pattern of fraction 8, showed only one pair of ions from the losses of fatty acid, corresponding to the loss of intact neutral fatty acids ([M + H-R1/2COOH]+) and of the acyl groups ([M + H-R1/2C=O]+) for each of the substituted fatty terminals (Figure 7). Moreover, ions [C10H22O5N]+ at m/z 236.15 (removal of both fatty acids from the intact molecule) and [C7H14O2N]+at m/z 144.10 (loss of glycerol and both fatty acids) are considered as the characteristic ions of DGTS (Y. Li, Y. Lou, T. Mu, J. Xu, C. Zhou, and X. Yan, "Simultaneous structural identification of diacylglyceryl-N-trimethylhomoserine (DGTS) and diacylglycerylhydroxymethyl-N,N,N-trimethyl-β-alanine (DGTA) in microalgae using dual Li+ /H+ adduct ion mode by ultra-performance liquid chromatography/quadrupole time-of-flight mass spectrometry," Rapid Communications in Mass Spectrometry, vol. 31, no. 5, pp. 457-468, 2017).

### Analysis of active fraction 8

Further Identification of bioactive components in fraction 8 was accomplished by measurements of higher concentrations to increase the detection limit (Figure 8). Identification of the detected molecules was done by comparison of the mass spectrometry fragmentation patterns with the NIST 05 MS-library (f-fit > 700; r-fit > 650) (A. van Vo et al., "Phosphorylation of Akt Mediates Anti-Inflammatory Activity of 1-p-Coumaroyl β-D-Glucoside Against Lipopolysaccharide-Induced Inflammation in RAW264.7 Cells," The Korean Journal of Physiology & Pharmacology: Official Journal of the Korean Physiological Society and the Korean Society of Pharmacology, vol. 18, no. 1, pp. 79-86, 2014), (K. Liu, L. Yan, G. Yao, and X. Guo, "Estimation of p-coumaric acid as metabolite of E-6-O-p-coumaroyl scandoside methyl ester in rat plasma by HPLC and its application to a pharmacokinetic study," Journal of Chromatography B, Analytical Technologies in the Biomedical and Life Sciences, vol. 831, 1-2, pp. 303-306, 2006).

Overall, 5 molecules were identified with different abundancies. Predominantly the identification of DGTS and Lyso-DGTS was achieved. Apart from the before detected molecule DGTS, second most abundant molecule was an ester-linked p-Coumaric acid which was also verified by using UV absorption measurements (600 nm).

Values shown in Table 9 should be regarded as a best estimate corresponding to the MS/MS Score.

**Table 9:**

| RT [min] | Mass [Da] | Molecular Formula | Molecule |
|---|---|---|---|
| 6.33 | 474.388 | C₂₆H₅₁NO₆ | Lyso-DGTS |
| 9.16 | 163.113 | C₉H₈O₃ | Ester-linked p-Coumaric acid |
| 10.01 | 281.272 | C₁₈H₃₅NO | Oleamide |
| 10.58 | 178.099 | C₇H₈N₄O₂ | Theophyline |
| 11.41 | 757.589 | C₄₄H₈₅NO₇ | DGTS |

Similarly, the *in vitro* kinase assay as described above has been used to investigate the direct inhibition of the IKKβ kinase by fraction 8 of the extract of *Chromochloris zofingiensis,* DGTS and again the whole extract of *Chromochloris zofingiensis* (Figure 9). The whole extract of *Chromochloris zofingiensis* as well as fraction 8 of said extract showed the same inhibitory activity as described before. DGTS showed no inhibition even at higher concentrations of that compound.

## Claims

1. An anti-inflammatory extract of soil microorganisms or a fraction thereof, wherein the microorganisms comprise one or more selected from *Chlorophyta, Xanthophyceae* or *Cyanobacteria.*

2. The extract or fraction thereof according to claim 1, wherein the soil microorganisms comprise one or more selected from *Xanthophyceae, Nostoc sp., Pseudochlorella sp., Coccomyxa sp., Neocystis brevis; Chromochloris sp., Chlorella saccharophila, Bracteacoccus sp., Dictyococcus varians, Muriella sp.,* or *Coelastrella sp.,* preferably from *Coccomyxa sp., Pseudochlorella subsphaerica, Nostoc sp.* or *Chromochloris zofingiensis.*

3. The extract or fraction thereof according to claims 1 or 2 obtainable by
a) extraction of the whole or part of the soil microorganisms with a solvent, and
b) optionally obtaining a fraction of the product of step a).

4. The extract or fraction thereof according to claim 3, wherein the solvent used in step a) comprises dichloromethane and/or methanol.

5. The extract or fraction thereof according to any one of the preceding claims, wherein the microorganisms have been cultivated under stress conditions.

6. The extract or fraction thereof according to claim 5, wherein the stress conditions include one or more of the following: light stress, oxidative stress, nutrient starvation/ deficiency, salinity stress, glucose addition, salt addition, or mixotrophic stress, preferably nitrogen starvation.

7. The extract or fraction thereof according to any one of the preceding claims, wherein the extract contains one or more of Lyso-Diacylglyceryl-N,N,N-trimethylhomoserine (Lyso-DGTS), p-Coumaric acid (ester-linked), oleamide (C₁₈H₃₅NO), theophylline (C₇H₈N₄O₂) or Diacylglyceryl-N,N,N-trimethylhomoserine (DGTS).

8. The extract or fraction thereof according to any one of the preceding claims, wherein the extract and/or the fraction thereof inhibits release of pro-inflammatory cytokines, preferably IL-10, IP-10, MCP-1 and RANTES.

9. The extract or fraction thereof according to any one of the preceding claims, wherein the extract and/or the fraction inhibits the IKK complex and/or the processes downstream of the IKK complex in the NF-_{K}B pathway, preferably the subunit IKKβ of the IKK complex in the NF-_{K}B pathway.

10. A composition comprising two or more of Lyso-DGTS, p-Coumaric acid (ester-linked), oleamide, theophylline or DGTS.

11. A pharmaceutical composition comprising an anti-inflammatory extract or a fraction thereof according to any one of claims 1 to 9 or a composition according to claim 10 as an active ingredient, optionally together with a suitable carrier substance and/or excipient.

12. An anti-inflammatory extract of soil microorganisms or a fraction thereof or a pharmaceutical composition comprising an anti-inflammatory extract of soil microorganisms or a fraction thereof or a composition comprising two or more of Lyso-DGTS, p-Coumaric acid (ester-linked), oleamide, theophylline or DGTS as an active ingredient for use as a medicament, wherein the microorganisms comprise one or more selected from *Chlorophyta, Xanthophyceae* or *Cyanobacteria.*

13. The extract or fraction thereof or the pharmaceutical composition for use according to claim 12 in the prophylaxis and/or treatment of inflammations and/or inflammatory diseases, preferably inflammations and/or inflammatory diseases of the skin and/or inflammations and/or inflammatory diseases involving NF-_{K}B.

14. The extract or fraction thereof or the pharmaceutical composition for use according to claims 12 or 13 wherein the inflammation and/or inflammatory disease is one or more of atopic dermatitis, psoriasis, contact dermatitis, allergic reactions such as chronic allergic reactions and allergic rhinitis, inflammatory rheumatic diseases, asthma bronchiale or colitis.

15. A non-pharmaceutical composition comprising an anti-inflammatory extract or fraction thereof according to any one of claims 1 to 9, preferably as a cosmetic composition or a food or drink supplement.
